# EUROPEAN PATENT SPECIFICATION

(11) **EP 0 358 774 B1**
(45) Date of publication and mention of the grant of the patent: **25.10.1995**
(21) Application number: 89902285.9
(22) Date of filing: 08.02.1989
(51) Int. Cl.: C07D 498/10

(54) **NOVEL SPIROOXAZINE COMPOUNDS**
SPIROOXAZIN-VERBINDUNGEN
NOUVEAUX COMPOSES DE SPIROOXAZINE

(30) Priority: 08.02.1988 JP 27327/88; 27.05.1988 JP 130833/88
(43) Date of publication of application: 21.03.1990
(73) Proprietor: TORAY INDUSTRIES, INC., Tokyo 103 (JP)
(72) Inventor: YAMAMOTO, Shinichi, Kyoto-shi Kyoto 607 (JP); TANIGUCHI, Takashi, Yasu-gun Shiga 520-23 (JP)
(74) Representative: Coleiro, Raymond
(86) International application number: JP8900124
(87) International publication number: WO8907104

(56) References cited:
- JP-A- 6 263 587

## Description

The present invention relates to a spiro-oxazine compound. More particularly, the present invention relates to a photochromic compound valuable in the fields of, for example, printing, optical recording, recording in general, clothing and decorating.

A spiropyran compound can be mentioned as a typical photochromic compound, and many photochromic spiropyran compounds are known [G. H. Brown, "Photochromism", Wiley Interscience, New York (1971)].

Spiropyran compounds, however, have a problem of poor fatigue resistance on repetition of coloration and decoloration.

A spiro-oxazine compound is known as a photochromic compound having an improved fatigue resistance. For example, JP-A-62-205185 discloses the following compound: 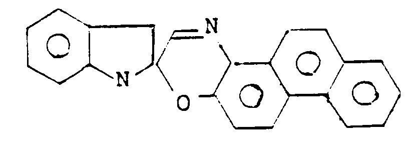
However, in the conventional spiro-oxazine compounds, the hue of the chromophoric seed is limited to violet-to-blue, and thus they provide only a limited selection of hues.

The present invention seeks to overcome the above-mentioned defects of the conventional technique, by providing a photochromic compound having an excellent fatigue resistance upon a repetition of coloration and decoloration and an abundance of hues.

According to the present invention, there is provided a spiro-oxazine compound represented by the following general formula (A) or (A'): 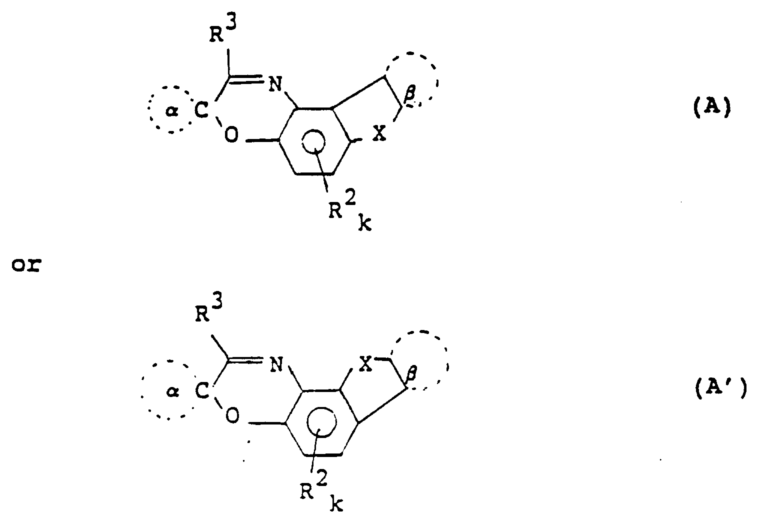
wherein the α ring is a ring selected from a 5-membered ring having one nitrogen atom, a 5-membered ring having one nitrogen atom and connected to a benzene ring or naphthalene ring and a 6-membered ring having one nitrogen atom, with the proviso that the nitrogen atom in the α ring is bonded to an organic group R⁰, expressed as > N-R⁰ (in which R⁰ is a substituent selected from an alkyl group having 1 to 20 carbon atoms, an alkenyl group having 2 to 20 carbon atoms, an aralkyl group having 7 to 20 carbon atoms and an aryl group having 6 to 19 carbon atoms); the β ring is a benzene ring or a naphthalene ring, X is a radical selected from O, S, Se and N-R¹ (in which R¹ is a substituent selected from a hydrogen atom, an alkyl group having 1 to 20 carbon atoms, an alkenyl group having 2 to 20 carbon atoms, an aralkyl group having 7 to 20 carbon atoms, an aryl group having 6 to 19 carbon atoms and an acyl group having 2 to 20 carbon atoms); each of R² and R³ is a substituent selected from a hydroxyl group, an amino group (as hereinafter defined), an alkoxy group having 1 to 20 carbon atoms, an aralkoxy group having 7 to 15 carbon atoms, an aryloxy group having 6 to 14 carbon atoms, an acyloxy group having 2 to 20 carbon atoms, an alkyl group having 1 to 20 carbon atoms, an alkenyl group having 2 to 20 carbon atoms, an aralkyl group having 7 to 15 carbon atoms, an aryl group having 6 to 14 carbon atoms, a halogen atom, a cyano group, a carboxyl group, a nitro group (not included in R³), an acyl group having 2 to 20 carbon atoms, an alkoxycarbonyl group, having 2 to 20 carbon atoms, a carbamoyl group (as hereinafter defined), a carbamoyloxy group (as hereinafter defined) and a sulphonic acid group (not included in R³), with the proviso that a hydrogen atom is included in R³; and k is 0 or an integer up to 2.

### BEST MODE FOR CARRYING OUT THE INVENTION

In the compound of the present invention represented by the general formula (A) or (A'), the α ring is a 5-membered or 6-membered ring having one nitrogen atom, or a 5-membered ring having one nitrogen atom and connected to a benzene ring or naphthalene ring. Specific examples are a pyrrolidine ring, a pyrrole ring, a piperidine ring, a tetrahydropyridine ring, a dihydropyridine ring, an indoline ring and a benzindoline ring.

The nitrogen atom contained in the α ring is bonded to an organic group R⁰, expressed as >N-R⁰.

Specific examples of the substituent R⁰ are linear alkyl groups having 1 to 20 carbon atoms, such as methyl, ethyl and octadecyl groups, branched alkyl groups having 3 to 20 carbon atoms, such as tert-butyl and 2-methylpentyl groups, cycloalkyl groups having 3 to 10 carbon atoms, such as cyclohexyl, norbornyl and adamantyl groups, alkenyl groups having 2 to 20 carbon atoms, such as vinyl, allyl, hexenyl, 1,3-butadienyl and isopropenyl groups, aralkyl groups having 7 to 20 carbon atoms, such as benzyl, phenethyl and (2-naphthyl)methyl groups, and aryl groups having 6 to 19 carbon atoms, such as phenyl and 2-naphthyl groups. R⁰ may be substituted. Specific examples of the substituent for R⁰ are a hydroxyl group, amino, dibenzylamino and (2-methacryloxyethyl)amino groups, methoxy, ethoxy and propoxy groups, acetoxy, benzoyloxy, acryloxy and methacryloxy groups, methyl, ethyl, trifluoromethyl and butyl groups, benzyl and 4-(2,3-epoxypropyl)phenethyl groups, phenyl and styryl groups, halogen atoms such as fluoro and chloro groups, a cyano group, a carboxyl group, a nitro group, acetyl and methacryl group, ethoxycarbonyl and 3,4-epoxybutyloxycarbonyl groups, carbamoyl, N-phenylcarbamoyl and N-(2-methacryloxy)ethylcarbamoyl groups, carbamoyloxy and [N-(acryloxy)propylcarbamoyl]oxy groups, and a sulphonic acid group and metal salts thereof (for example, sodium and lithium salts).

The β ring, which is included in the general formula (A) or (A') as well as the α ring, is a benzene ring or a naphthalene ring.

Other constituents included in the general formula (A) or (A') will now be described.

X is a radical selected from O, S, Se and N-R¹, in which R¹ is a substituent selected from a hydrogen atom, an alkyl group having 1 to 20 carbon atoms, an alkenyl group having 2 to 20 carbon atoms, an aralkyl group having 7 to 20 carbon atoms, an aryl group having 6 to 19 carbon atoms and an acyl group having 2 to 20 carbon atoms. R¹ may be substituted. Specific examples of substituents on any of the alkyl, alkenyl, aralkyl and aryl groups of R¹ are those exemplified above with respect to R⁰, and, in particular, the acyl group may be an acetyl group or a methacryl group.

In order to obtain a compound having an excellent repetition durability and having a plurality of absorption wavelengths of the chromophoric seed, X is preferably O. Furthermore, to obtain a chromophoric seed of a bright red hue, X is preferably N-R¹.

Specific examples of R² and R³ are a hydroxyl group, an amino group selected from unsubstituted amino, dimethylamino, dibenzylamino and (2-methacryloxyethyl)amino groups, alkoxy groups having 1 to 20 carbon atoms, such as methoxy, ethoxy, propoxy and butoxy groups, aralkoxy groups having 7 to 15 carbon atoms, such as benzyloxy and phenethyloxy groups, aryloxy groups having 6 to 14 carbon atoms, such as phenoxy and naphthyloxy groups, acyloxy groups having 2 to 20 carbon atoms, such as benzoyloxy, acryloxy and methacryloxy groups, alkyl groups having 1 to 20 carbon atoms, such as methyl, ethyl, propyl, butyl, tert-butyl, glycidyl and octadecyl groups, alkenyl groups having 2 to 20 carbon atoms, such as vinyl, allyl, isopropenyl, 1,3-butadienyl and 9,12-octadecadienyl groups, aralkyl groups having 7 to 15 carbon atoms, such as benzyl, phenethyl and naphthylmethyl groups, aryl groups having 6 to 14 carbon atoms, such as phenyl and naphthyl groups, halogen atoms such as chloro, fluoro and bromo, a cyano group, a carboxyl group, a nitro group (not included in R³), acyl groups having 2 to 20 carbon atoms, such as acetyl, methacryl and acryl groups, alkoxycarbonyl groups having 2 to 20 carbon atoms, such as ethoxycarbonyl and 3,4-epoxybutyloxycarbonyl groups, carbamoyl groups selected from unsubstituted carbamoyl, N-phenylcarbamoyl and N-(2-methacryloxy)-ethylcarbamoyl groups, carbamoyloxy groups selected from carbamoyloxy, [N-(3-acryloxy)propylcarbamoyl]oxy and [N-(methacryloxy)propylcarbamoyl]oxy groups, and a sulphonic acid group (not included in R³) and metal salts thereof (for example, sodium and lithium salts). Furthermore, a hydrogen atom is included in R³.

Note, k is 0 or an integer up to 2, and where k is 2, the two R² groups may be the same as or different from one another.

Specific examples of the spiro-oxazine compound of the present invention represented by the general formula (A) or (A') are the following compounds.
(a) Spiro-oxazine compounds represented by the following general formula (B) or (B'): 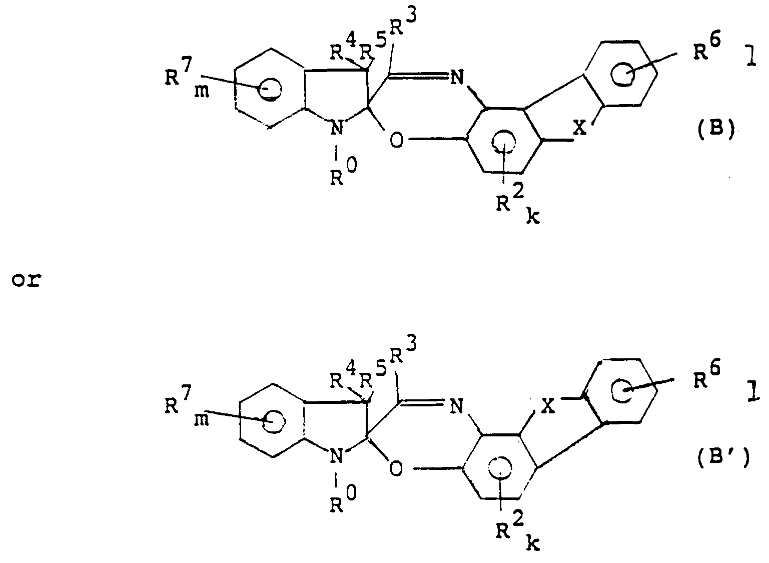 wherein R⁰, R², R³, X and k are as defined in the general formula (A) or (A'), each of R⁴ and R⁵ independently is a substituent selected from an alkyl group having 1 to 20 carbon atoms, an aralkyl group having 7 to 20 carbon atoms and an aryl group having 6 to 19 carbon atoms, or R⁴ and R⁵ together form a cycloalkyl group having 3 to 10 carbon atoms with the carbon atom at the 3'-position, each of R⁶ and R⁷ is as defined in the general formula (A) or (A') with respect to R² and may be a substituent selected from a hydroxyl group, an amino group (as hereinbefore defined), an alkoxy group having 1 to 20 carbon atoms, an aralkoxy group having 7 to 15 carbon atoms, an aryloxy group having 6 to 14 carbon atoms, an acyloxy group having 2 to 20 carbon atoms, an alkyl group having 1 to 20 carbon atoms, an alkenyl group having 2 to 20 carbon atoms, an aralkyl group having 7 to 15 carbon atoms, an aryl group having 6 to 14 carbon atoms, a halogen atom, a cyano group, a carboxyl group, a nitro group, an acyl group having 2 to 20 carbon atoms, an alkoxycarbonyl group having 2 to 20 carbon atoms, a carbamoyl group (as hereinbefore defined), a carbamoyloxy group (as hereinbefore defined) and a sulphonic acid group, and each of l and m is 0 or an integer up to 4.
(b) Spiro-oxazine compounds represented by the following general formula (C) or (C'): 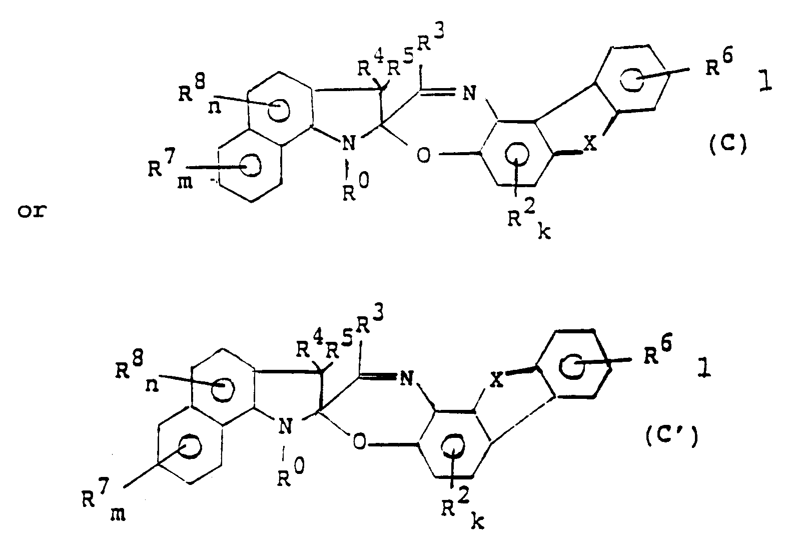 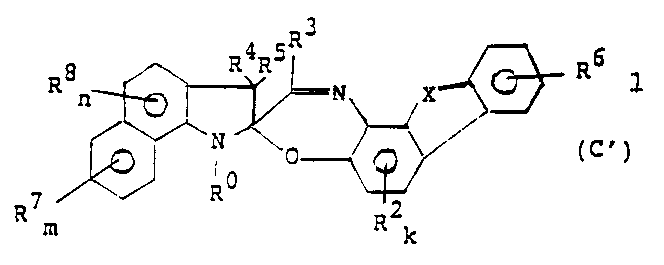 wherein R⁰, R², R³, X and k are as defined in the general formula (A) or (A'), R⁴, R⁵, R⁶, R⁷, l and m are as defined in the general formula (B) or (B'), R⁸ is as defined in the general formula (A) or (A') with respect to R² and may be a substituent selected from a hydroxyl group, an amino group (as hereinbefore defined), an alkoxy group having 1 to 20 carbon atoms, an aralkoxy group having 7 to 15 carbon atoms, an aryloxy group having 6 to 14 carbon atoms, an acyloxy group having 2 to 20 carbon atoms, an alkyl group having 1 to 20 carbon atoms, an alkenyl group having 2 to 20 carbon atoms, an aralkyl group having 7 to 15 carbon atoms, an aryl group having 6 to 14 carbon atoms, a halogen atom, a cyano group, a carboxyl group, a nitro group, an acyl group having 2 to 20 carbon atoms, an alkoxycarbonyl group having 2 to 20 carbon atoms, a carbamoyl group (as hereinbefore defined) a carbamoyloxy group (as hereinbefore defined) and a sulfonic acid group, and n is 0 or an integer up to 2.
(c) Spiro-oxazine compounds represented by the following general formula (D) or (D'): 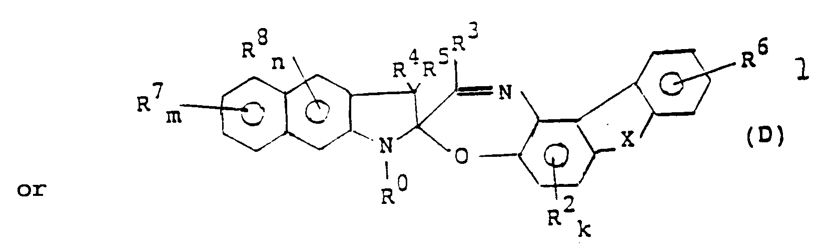 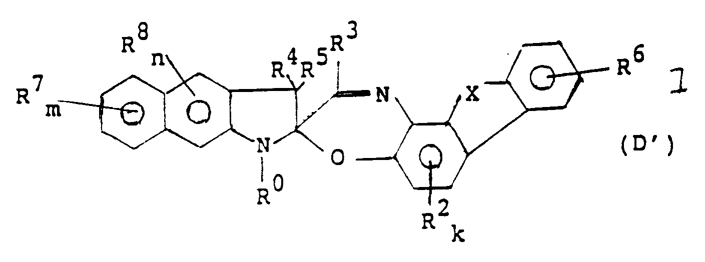 wherein R⁰, R², R³, X and k are as defined in the general formula (A) or (A'), R⁴ R⁵, R⁶, R⁷, l and m are as defined in the general formula (B) or (B'), and R⁸ and n are as defined in the general formula (C) or (C').
(d) Spiro-oxazine compounds represented by the following general formula (E) or (E'): 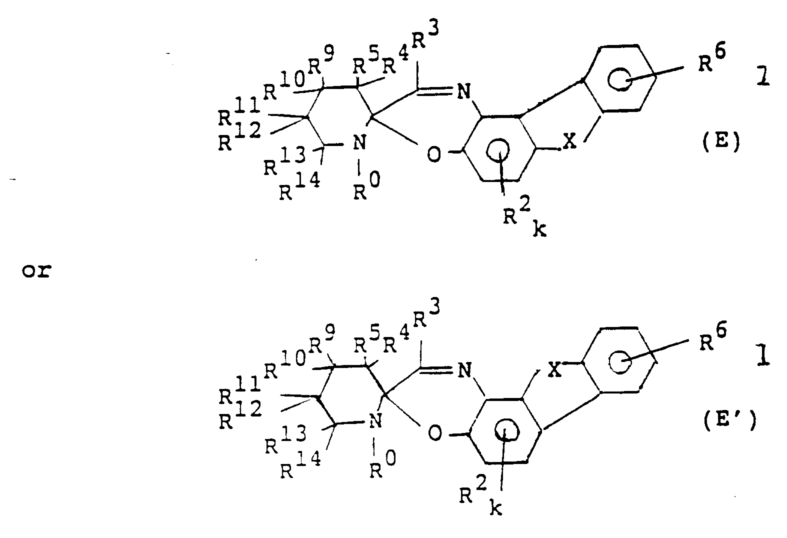 wherein R⁰, R², R³, X and k are as defined in the general formula (A) or (A'), R⁴, R⁵, R⁶ and l are as defined in the general formula (B) or (B'), and each of R⁹, R¹⁰, R¹¹, R¹², R¹³ and R¹⁴ is a substituent selected from a hydrogen atom, an alkyl group having 1 to 20 carbon atoms, an aralkyl group having 7 to 20 carbon atoms and an aryl group having 6 to 19 carbon atoms.
(e) Spiro-oxazine compounds represented by the general formula (F) or (F'): 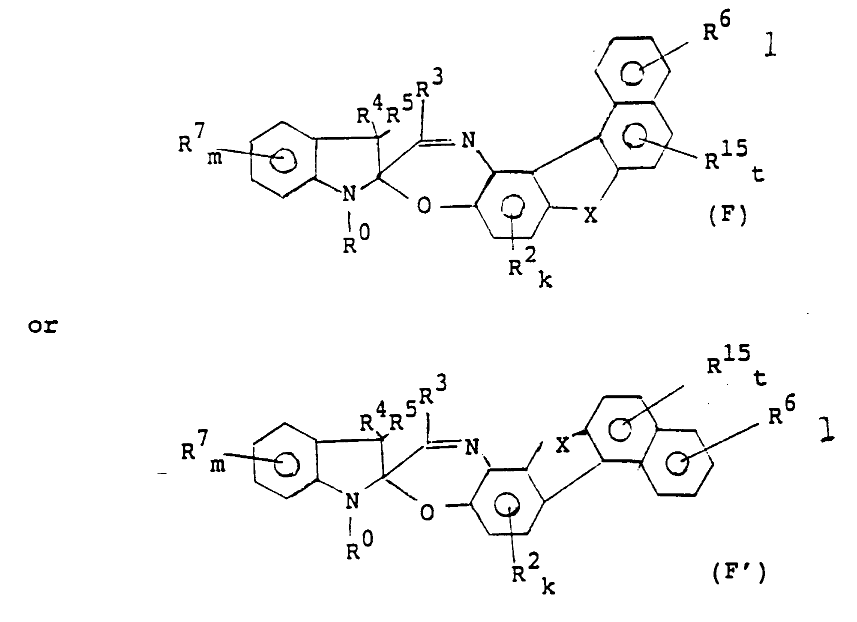 wherein R⁰, R², R³, X and k are as defined in the general formula (A) or (A'), R⁴, R⁵, R⁶, R⁷, l and m are as defined in the general formula (B) or (B'), R¹⁵ is a substituent selected from a hydroxyl group, an amino group, an alkoxy group having 1 to 20 carbon atoms, an aralkoxy group having 7 to 15 carbon atoms, an aryloxy group having 6 to 14 carbon atoms, an acyloxy group having 2 to 20 carbon atoms, an alkyl group having 1 to 20 carbon atoms, an alkenyl group having 2 to 20 carbon atoms, an aralkyl group having 7 to 15 carbon atoms, an aryl group having 6 to 14 carbon atoms, a halogen atom, a cyano group, a carboxyl group, a nitro group, an alkoxycarbonyl group having 2 to 20 carbon atoms, a carbamoyl group, a carbamoyloxy group and a sulphonic acid group, and t is 0 or an integer up to 2.

In the spiro-oxazine compounds represented by the above-mentioned general formulae (B), (B'), (C), (C'), (D) and (D'), specific examples of R⁴ and R⁵, which are independent from each other, are linear alkyl groups having 1 to 20 carbon atoms, such as methyl, ethyl and octadecyl groups, branched alkyl groups having 3 to 20 carbon atoms, such as a 2-methylpentyl group, cycloalkyl groups having 3 to 10 carbon atoms, such as a cyclohexyl group, aralkyl groups having 7 to 20 carbon atoms, such as benzyl and phenethyl groups and aryl groups having 6 to 19 carbon atoms, such as a phenyl group, and specific examples of the group formed by combined R⁴ and R⁵ together with the carbon at the 3'-position are cycloalkyl groups having 3 to 10 carbon atoms, such as cyclohexyl, norbornyl and adamantyl groups.

R⁴ and R⁵ may be substituted. The substituent for R⁴ and R⁵ can be any of those exemplified above as the substituent for R⁰.

R⁶, R⁷ and R⁸ are as defined in the general formula (A) or (A') with respect to R². l, m and n are as defined above, and if any of l, m and n is 2 or more, each of corresponding groups R⁶, R⁷ or R⁸ may be the same as or different from each other.

In the spiro-oxazine compounds represented by the above-mentioned general formula (E) or (E'), R⁰, R², R³, R⁴, R⁵, R⁶, X, k and l are as defined in the above-mentioned general formulae (A), (A'), (B), (B'), (C), (C'), (D) and (D'), and R⁹, R¹⁰, R¹¹, R¹², R¹³ and R¹⁴ are selected from a hydrogen atom and the same alkyl, aralkyl and aryl groups as exemplified above with respect to R² in the general formula (A) or (A'). All of these groups may be the same or different.

In the spiro-oxazine compounds represented by the general formula (F) or (F'), groups other than R¹⁵ are as mentioned in the other general formulae, and R¹⁵ is selected from certain of the groups exemplified above with respect to R⁰ in the general formula (A) or (A'). If t is 2, each R¹⁵ group may be the same as or different from the other.

As is apparent from the foregoing description, the spiro-oxazine compound of the present invention is characterized in that the spiro-oxazine compound comprises not only the above-mentioned α ring but also an oxazine ring, a benzene ring, a heterocyclic 5-membered ring and the β ring. Especially, a compound in which the benzene ring, heterocyclic 5-membered ring and β ring are consecutively connected, R³ is a hydrogen atom and X is O, S or Se, has absorptions in both of the short wavelength region (less than 550 nm) and long wavelength region (more than 600 nm) of the visible ray region, and even a compound developing a green color can be obtained. Furthermore, if R³ has a substituent other than hydrogen or X is N-R¹, a compound developing a red color can be obtained. Accordingly, the spiro-oxazine compound of the present invention has an abundance of chromophoric seed. Moreover, the spiro-oxazine compound of the present invention has an excellent fatigue resistance to the repetition of coloration and decoloration.

The general formulae (A) and (A') in the present invention are isomeric to each other, and an appropriate isomer is selected according to the intended object in view of the kinds of substituents and the desired photochromic characteristics.

The compound of the present invention represented by the formula (A) or (A') is prepared, for example, according to any of the following processes.

According to the first process, a methylene compound represented by the following general formula (I): 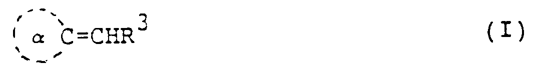
is reacted with a nitroso compound represented by the following general formula (II) or (II'): 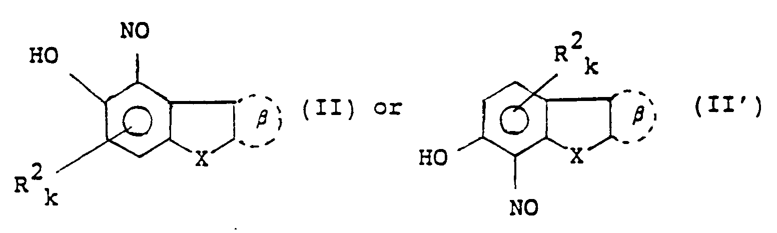
to prepare a compound represented by the formula (A) or (A').

According to the second process, a compound represented by the following general formula (III): 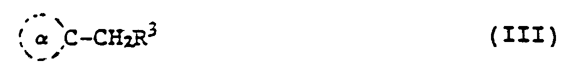
is reacted with a quaternarizing agent R⁰-Y (Y stands for an anionic leaving group), a basic substance and a compound represented by the general formula (II) or (II') in an optional order to obtain a compound represented by the formula (A) or (A').

According to the third process, a reaction product between a compound represented by the formula (I) and nitrous acid is reacted with an amino compound represented by the following general formula (IV) or (IV'): 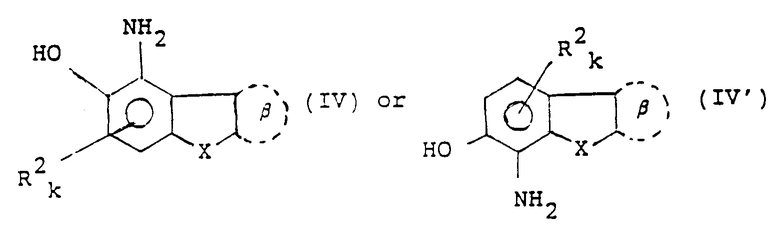
to prepare a compound represented by the formula (A) or (A').

According to the fourth process, a reaction product between a compound represented by the formula (III) and nitrous acid is reacted with a quaternarizing agent R⁰-Y, a basic substance and a compound represented by the general formula (IV) or (IV') in an optional order to prepare a compound represented by the formula (A) or (A').

According to the fifth process, a reaction product between a compound represented by the following general formula (V): 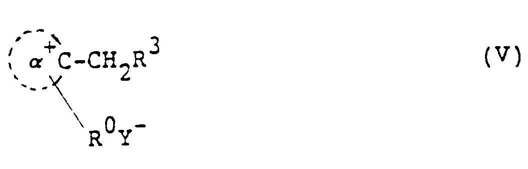
and nitrous acid is reacted with a basic substance and a compound represented by the general formula (IV) or (IV') in an optional order to prepare a compound represented by the formula (A) or (A').

As the purification method in the preparation process, preferably recrystallization using various solvents, column chromatography separation using a silica column or the like, solvent extraction and active carbon treatment are adopted.

The spiro-oxazine compound of the present invention is preferably used in combination with an optically transparent resin. The resin can be, for example, a diethylene glycol bis-allylcarbonate polymer, a (meth)acrylic polymer, a copolymer thereof, a cellulose, polyvinyl acetate, polyvinyl alcohol, polyvinyl butyral, a polyester resin, polycarbonate, polystyrene, a copolymer thereof, an epoxy resin, a (halogenated) bisphenol A di(meth)acrylate polymer, a copolymer thereof, a (halogenated) bisphenol A urethane-modified di(meth)acrylate polymer, a copolymer thereof, a nylon resin and polyurethane.

If the spiro-oxazine compound of the present invention has an alkenyl group, that is, a polymerizable functional group such as a methacryloxy group or a vinyl group, by copolymerization of this spiro-oxazine compound with other polymerizable compound such as an acrylic monomer, a styrene type monomer or a vinyl acetate type monomer, the spiro-oxazine compound can be integrated with a polymer resin of the other polymerizable compound. If the spiro-oxazine compound is thus integrated by the copolymerization, a preferred photochromic material having an excellent durability of the spiro-oxazine compound can be obtained.

Compounds of the general formulae (A) to (F) and (A') to (F') having polymerizable functional groups at R⁰, R², R⁶, R⁷ and R¹⁵ are preferable from the viewpoint of durability. Compounds having a polymerizable functional group at R⁰, which is introduced through a methylene chain having at least 3 carbon atoms, are especially preferred. A methacryloxy group is especially preferred as the polymerizable functional group, because the radical polymerization is easily accomplished and decomposition is prevented during the polymerization.

If the compound of the present invention has a polymerizable functional group, the compound can be used in the form of not only a copolymer as mentioned above but also a homopolymer.

The photochromic compound of the present invention can be preferably used as an optical element having photochromic characteristics in the form of a combination with a resin, a polymer of the compound or a combination of this polymer with other resin. As the optical elements, there can be mentioned sunglass lenses, ski goggles and protective glass lenses. Furthermore, the photochromic compound of the present invention can be preferably used for curtaining, clothing, automobile windows such as a front glass or sun roof, toys, toilet articles and writing tools.

As the method for combining the photochromic compound of the present invention, various methods can be adopted, for example, a dyeing method, a casting method and a polymer solution-coating method. Instead of the polymer solution-coating method, a method can be adopted in which an emulsion is formed and the emulsion is coated by screen printing. Moreover, various printing methods, for example, a gravure printing method, can be adopted. As the coating method, various coating methods can be adopted, such as a dip coating method, a spin coating method and a roll coating method.

In many cases, the chromophoric seed of the compound of the present invention has at least two absorption peaks, and therefore, the compound of the present invention is valuable as a material for a rewritable optical disk.

The amount of the compound of the present invention to be combined with the resin should be determined according to the object and the application method, but from the viewpoint of visual sensitivity, preferably the amount of the compound of the present invention is 0.01 to 20% by weight based on the resin.

To improve the repetition durability of the photochromic compound of the present invention, preferably oxygen and water are intercepted when the compound of the present invention is used. Furthermore, known additives can be used to further improve the durability. For example, there can be used a single t oxygen quencher represented by a nickel salt, an antioxidant represented by a hindered amine or a polymer thereof and an ultraviolet absorber having no influence on the photochromic characteristics.

The present invention will now be described in detail with reference to the following examples that by no means limit the scope of the invention.

### Example 1

### (1) Synthesis of 1-nitroso-2-hydroxydibenzofuran

In 100 g of pyridine was dissolved 10 g of 2-hydroxydibenzofuran, and the solution was cooled to 0°C. Then, 60 g of a 20% aqueous solution of sodium nitrite was added to the above solution and 100 g of a 30% aqueous solution of sulfuric acid was added dropwise to the mixture while stirring for 10 minutes. After the dropwise addition, the mixture was stirred at 0°C for 1 hour and then filtered. The recovered solid was washed with water and dried to obtain 11 g of a red crystal of 1-nitroso-2-hydroxydibenzofuran.

### (2) Results of analysis

Elemental analysis values:

| | Found Value (%) | Calculated Value (%) |
|---|---|---|
| C | 67.7 | 67.7 |
| H | 3.2 | 3.3 |
| N | 6.3 | 6.6 |

NMR (protons other than OH): 6.7 ppm (d, 1H), 7.4 ppm (t, 1H), 7.5 ppm (t, 1H), 7.6 ppm (d, 1H), 7.7 ppm (d, 1H), 8.3 ppm (d, 1H)

### (3) Synthesis of spiro-oxazine of following formula (G):

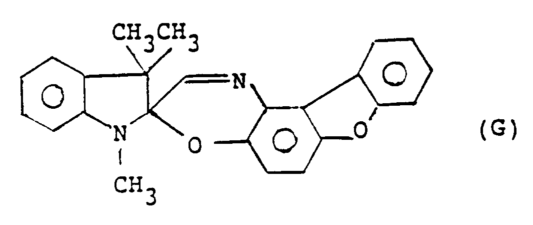

In 100 ml of absolute ethanol were dissolved 10 g of 1,3,3-trimethyl-2-methylene-indoline and 10 g of 1-nitroso-2-hydroxydibenzofuran, and reaction was carried out at the reflux temperature for 1 hour. After the reaction, the reaction mixture was concentrated and subjected to column chromatography separation using silica gel as the supporting carrier and methylene chloride as the developing solvent. Distillation of methylene chloride gave a pink solid, and recrystallization from methanol gave a white crystalline spiro-oxazine of the formula (G).

### (4) Results of analysis

Elemental analysis values:

| | Found Value (%) | Calculated Value (%) |
|---|---|---|
| C | 78.8 | 78.3 |
| H | 5.8 | 5.4 |
| N | 7.2 | 7.6 |

NMR: 1.4 ppm (6H), 2.8 ppm (3H), 6.6-8.5 ppm (11H) Melting point: 158°C

### (5) Application

A coating solution was prepared by dissolving 0.1 g of the spiro-oxazine of the formula (G) in 100 g of a 10% solution of polyvinyl butyral in butanol, and the coating solution was coated on two glass sheets. The coated glass sheets were dried and piled so that the resin-coated surfaces confronted each other, and the piled glass sheets were heated.

When the so-prepared photochromic laminated glass was irradiated with ultraviolet rays, the laminated glass became green. If the light was removed and the laminated glass was allowed to stand in the dark, the laminated glass became colorless again. When the laminated glass was irradiated with light for 20 hours in a fadeometer and the light resistance was examined, it was found that the photochromic characteristics were the same as those before the placement in the fade meter, and it was confirmed that the fatigue resistance was excellent. When the absorption characteristics at the time of coloration in methanol were examined, it was found that absorption peaks λₘₐₓ appeared at 460 nm and 632 nm.

### Example 2

### (1) Synthesis of spiro-oxazine of following formula (S):

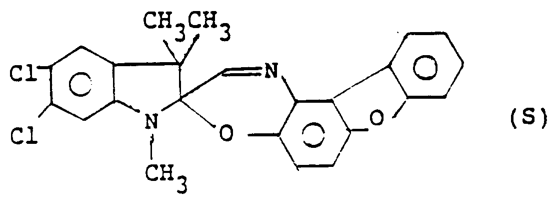

A white crystalline spiro-oxazine of the formula (S) was obtained in the same manner as described in Example 1 except that 1,3,3-trimethyl-2-methylene-5,6-dichloroindoline was used instead of 1,3,3-trimethyl-2-methyleneindoline.

### (2) Results of analysis

Elemental analysis values:

| | Found Value (%) | Calculated Value (%) |
|---|---|---|
| C | 66.1 | 65.9 |
| H | 3.8 | 4.1 |
| N | 6.5 | 6.4 |

Melting point: 204 - 205°C
NMR: 1.5 ppm (6H), 2.8 ppm (3H), 6.4-8.4 ppm (9H)

### (3) Application

A laminated glass was prepared in the same manner as described in Example 1 using the so-obtained compound. When the laminated glass was irradiated with ultraviolet rays, the laminated glass became green, and if the light was removed and the laminated glass was allowed to stand in the dark, the laminated glass became colorless again. When the laminated glass was irradiated with light for 20 hours in a fadeometer and the light resistance was examined, it was found that the photochromic characteristics were the same as those before the placement in the fadeometer and the light resistance was excellent.

### Example 3

### (1) Synthesis of spiro-oxazine of formula (H)

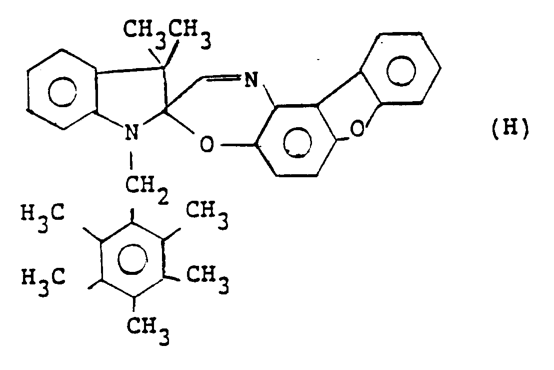

In 100 ml of benzene were dissolved 10 g of 2,3,3-trimethylindolenine and 15 g of 2,3,4,5,6-pentamethylbenzyl chloride, and the solution was refluxed for 2 hours. Then, the temperature of the reaction liquid was lowered to about 50°C, and 3 g of triethylamine and 10 g of 1-nitroso-2-hydroxydibenzofuran were added to the reaction liquid. Reaction was carried out at the reflux temperature for 1 hour. Then, the purification was carried out in the same manner as described in Example 1 to obtain a white crystalline spiro-oxazine of the formula (G).

### (2) Results of analysis

Elemental analysis values:

| | Found Value (%) | Calculated Value (%) |
|---|---|---|
| C | 81.9 | 81.7 |
| H | 6.8 | 6.6 |
| N | 5.2 | 5.4 |

NMR: 1.4 ppm (6H), 2.2 ppm (15H), 4.3 ppm (2H), 6.4-8.5 ppm (11H)

### (3) Application

A laminated glass was prepared in the same manner as described in Example 1 by using the so-prepared compound. When the absorption characteristics of the laminated glass at the time of coloration were examined, it was found that the absorption peaks λₘₐₓ appeared at 460 nm and 632 nm.

### Examples 4 to 11

### (1) The following compounds having substituents at the positions a to g were prepared in the same manner as described in Example 3.

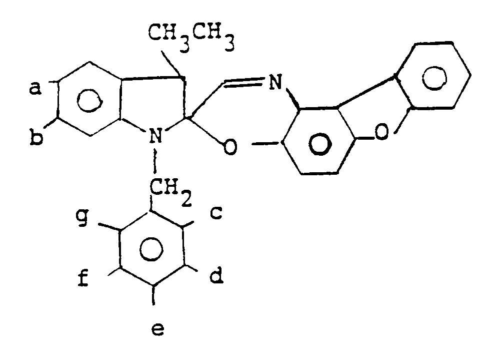

The compound of Example 4 had Cl at each of the positions a, b, c and e.

The compound of Example 5 had F at each of the positions c, d, e, f and g.

The compound of Example 6 had OCH₃ at each of the positions a and c and NO₂ at the position e.

The compound of Example 7 had a methacryloxy group at the position a.

The compound of Example 8 had a methoxy group at the position a and a vinyl group at the position e.

The compound of Example 9 had H at each of the positions a to g.

The compound of Example 10 had Cl at each of the positions c and e.

The compound of Example 11 had CH₃ at the position d.

### (2) Results of analysis

Elemental analysis values:

| | C (%) | H (%) | N (%) |
|---|---|---|---|
| Example 4 | 62.2 (61.9) | 3.5 (3.4) | 4.7 (4.8) |
| Example 5 | 67.5 (67.4) | 3.9 (3.6) | 5.0 (5.2) |
| Example 6 | 70.3 (69.9) | 5.2 (4.9) | 7.6 (7.7) |
| Example 7 | 77.1 (77.3) | 5.2 (5.3) | 5.3 (5.3) |
| Example 8 | 79.0 (79.2) | 5.3 (5.6) | 5.7 (5.6) |
| Example 9 | 81.1 (81.1) | 5.3 (5.4) | 6.3 (6.3) |
| Example 10 | 70.2 (70.2) | 4.0 (4.3) | 5.5 (5.5) |
| Example 11 | 81.0 (81.2) | 5.8 (5.7) | 6.0 (6.1) |

Each value is a measured value but each of the parenthesized values is a calculated value.
NMR:
Example 9: 1.4 ppm (6H), 4.4 ppm (4H), 6.3-8.4 ppm (16H)
Example 10: 1.4 ppm (6H), 4.5 ppm (2H), 6.3-8.4 ppm (14H)
Example 11: 1.4 ppm (6H), 2.3 ppm (3H), 4.5 ppm (2H), 6.2-8.5 ppm
Melting point:
Example 9: 157 - 158°C
Example 10: 150°C

### (3) Application

Laminated glass samples were prepared in the same manner as described in Example 1 by using the compounds of Examples 4 to 6. Each of the laminated glass samples became green under irradiation with ultraviolet rays.

In 20 g of toluene were dissolved 1 g of the compound of Example 7 and 10 g of n-butyl methacrylate, and solution polymerization was carried out by using azobisisobutyronitrile as the polymerization initiator. When the polymer solution was coated on a slide glass, a transparent coating film was obtained. When the glass sheet was irradiated with ultraviolet rays, the glass sheet became green, and when the light was removed and the glass sheet was allowed to stand in the dark, the glass sheet became colorless again.

A glass sheet was similarly prepared using the compound of Example 8, and the glass sheet became green under irradiation with ultraviolet rays.

A coating solution formed by dissolving 0.1 g of the compound of Example 9 in a 10% solution of an acrylic polymer in toluene was coated on two glass sheets. The glass sheets were dried and piled so that the resin-coated surfaces confronted each other, and the laminate then heated.

When the so-prepared photochromic laminated glass was irradiated with ultraviolet rays, the laminated glass became green, and when the light was removed and the laminated glass was allowed to stand in the dark, the laminated glass became colorless again. When the laminated glass was irradiated with light for 20 hours in a fadeometer and the light resistance was examined, it was found that the photochromic characteristics were the same as those before the placement in the fadeometer and the light resistance was excellent. When the absorption characteristics at the time of coloration were examined, it was found that the absorption peaks λₘₐₓ appeared at 486 nm and 632 nm.

A laminated glass was prepared in the same manner as described above using the compound of Example 10, and when the absorption characteristics at the time of coloration were examined, it was found that the absorption peaks λₘₐₓ appeared at 486 nm and 628 nm.

A coating solution comprising 0.1 g of the compound of Example 11, 0.03 g of a hindered amine type light stabilizer (LA-57 supplied by Adeca-Argus), 0.04 g of a hindered amine type light stabilizer (LA-77 supplied by Adeca-Argus), 0.02 g of a hindered phenol type antioxidant (AO-60 supplied by Adeca-Argus), 0.01 g of di(2-ethylhexyl) adipate as a plasticizer, 0.1 g of dimethyl diphenate and 100 g of a 10% solution of polyvinyl butyral in butanol was coated on two glass sheets. The coated glass sheets were dried and piled so that the resin-coated surfaces confronted each other, and the laminate then heated. When the so-prepared photochromic laminated glass was irradiated with ultraviolet rays, the laminated glass became green, and when the light was removed and the laminated glass was allowed to stand in the dark, the laminated glass became colorless again. When the laminated glass was irradiated with light for 20 hours in a fadeometer and the light resistance was examined, it was found that the light resistance was good.

### Example 12

### (1) Synthesis of spiro-oxazine of following formula (I):

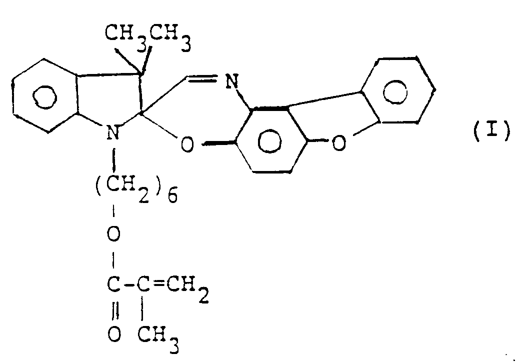

To a solution comprising 15 g of 1-(6-hydroxyhexyl)-2,3,3-trimethylindolenium iodide, 10 g of triethylamine and 100 ml of methylene chloride was added dropwise 15 g of methacryloyl chloride. After the dropwise addition, the mixture was stirred for 1 hour. Then, 100 ml of water was added to the mixture, and the mixture was extracted with methylene chloride. The methylene chloride layer was concentrated to obtain 11 g of 1-(6-methacryloxy)hexyl-2-methylene-3,3-dimethyl-indoline. The reaction was carried out in the same manner as described in Example 1 except that the so-obtained compound was used instead of 1,3,3-trimethyl-2-methylene-indoline, whereby a yellow liquid of the spiro-oxazine of the formula (I) was obtained.

### (2) Results of analysis

Elemental analysis values:

| | Found Value (%) | Calculated Value (%) |
|---|---|---|
| C | 75.8 | 75.9 |
| H | 6.4 | 6.5 |
| N | 5.4 | 5.4 |

IR: 1720 cm⁻¹
NMR: 1.2-2.0 ppm (17H), 3.2 ppm (2H), 4.1 ppm (2H), 5.5 ppm (1H), 6.1 ppm (1H), 6.6-8.5 ppm (11H)

### (3) Application

In the same manner as described in Example 7, a coated glass sheet was prepared using the spiro-oxazine of the formula (I). When the glass sheet was irradiated with ultraviolet rays, the glass sheet became green, and when the light was removed and the glass sheet was allowed to stand in the dark, the glass sheet became colorless again. When the absorption characteristics at the time of coloration were examined, it was found that the absorption peaks λₘₐₓ appeared at 486 nm and 633 nm.

When the glass sheet was subjected to the durability test where the glass sheet was irradiated with light for 20 hours in a fadeometer, no substantial degradation was observed, and it was confirmed that the glass sheet had a good durability.

### Example 13

### (1) Synthesis of spiro-oxazine compound of formula (J)

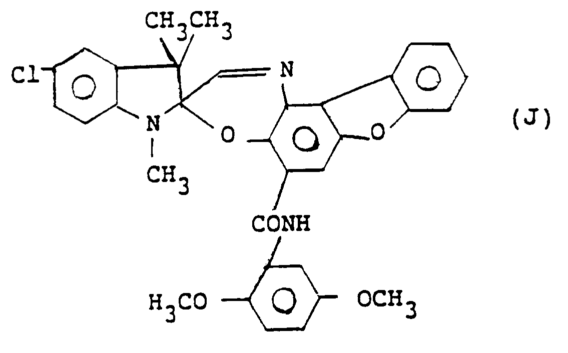

The above compound was synthesized in a manner similar to the manner adopted in Example 1.

### (2) Results of analysis

Elemental analysis values:

| | Found Value (%) | Calculated Value (%) |
|---|---|---|
| C | 72.2 | 72.4 |
| H | 5.4 | 5.3 |
| N | 7.4 | 7.7 |

IR: 3380 cm⁻¹, 3340 cm⁻¹, 1710 cm⁻¹, 1540 cm⁻¹, 1250 cm⁻¹, 1025 cm⁻¹

### (3) Application

The spiro-oxazine of the formula (J) was dissolved in methyl methacrylate at a concentration of 0.5% by weight, and cast polymerization was carried out using azobisisobutyronitrile as the polymerization initiator to obtain a polymethyl methacrylate sheet in which the compound of the formula (J) was incorporated.

When the sheet was irradiated with ultraviolet rays, the sheet became green, and when the light was removed and the sheet was allowed to stand in the dark, the sheet promptly became colorless again. When the absorption characteristics at the time of coloration were examined, it was found that the absorption peaks λₘₐₓ appeared at 490 nm and 706 nm. This sheet became green even at 50°C under irradiation with ultraviolet rays, and the coloration-possible temperature was higher than 50°C.

### Example 14

### (1) Synthesis of 2,3,3-trimethylbenz(g)indolenine

To 10 g of 1-naphthylhydrazine hydrochloride and 30 g of methyl isopropyl ketone was added dropwise 5 g of sulfuric acid, and reaction was carried out at the reflux temperature for 2 hours. The reaction mixture was neutralized with a 20% aqueous solution of sodium hydroxide and extracted with water/ether, and the ether layer was concentrated to obtain 8 g of 2,3,3-trimethylbenz(g)indolenine.

### (2) Synthesis of 1,2,3,3-tetramethylbenz(g)indolenium iodide

A solution comprising 8 g of 2,3,3-trimethylbenz(g)indolenine and 25 g of methyl iodide was reacted at the reflux temperature for 30 minutes. The formed precipitate was recovered by filtration and washed with acetone to obtain 12 g of a white crystal.

### (3) Synthesis of spiro-oxazine of following formula (K):

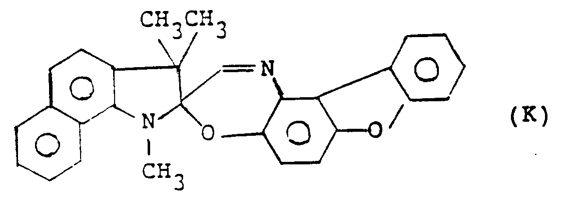

A solution of 12 g of 1,2,3,3-tetramethylbenz(g)indolenium iodide and 10 g of 1-nitroso-2-hydroxydibenzofuran in 50 ml of isopropanol was heated to 50°C, and 5 g of piperidine was added to the solution. Then, reaction was carried out at the reflux temperature for 1 hour. The reaction mixture was concentrated and subjected to the column chromatography separation using silica gel as the supporting carrier and toluene as the developing solvent. Distillation of the solvent gave a pink solid. When this solid was recrystallized from butanol, a light yellow crystal of the compound of the formula (K) was obtained.

### (4) Results of analysis

Elemental analysis values:

| | Found Value (%) | Calculated Value (%) |
|---|---|---|
| C | 80.5 | 80.4 |
| H | 5.6 | 5.3 |
| N | 6.4 | 6.7 |

NMR: 1.4 ppm (6H), 3.4 ppm (3H), 6.4-8.7 ppm (13H)

### (5) Application

A laminated glass was prepared in the same manner as described in Example 9 using the compound of the formula (K). When the laminated glass was irradiated with ultraviolet rays, the laminated glass became green, and when the light was removed and the laminated glass was allowed to stand in the dark, the laminated glass became colorless again. When the laminated glass was irradiated with light for 20 hours in a fadeometer and the light resistance was examined, it was found that the photochromic characteristics were the same as those before the placement in the fadeometer and the fatigue resistance was excellent. When the absorption characteristics at the time of coloration were examined, it was found that the absorption peaks λₘₐₓ appeared at 465 nm and 650 nm.

### Examples 15 to 18

Compounds of the following formula having substituents at the positions a and b were synthesized in a manner similar to the manner adopted in Example 7: 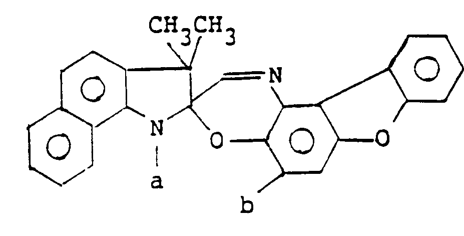
The compound of Example 15 had 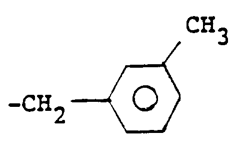
at the position a.

The compound of Example 16 had -CH₃ at the position a and -CO₂H at the position b.

The compound of Example 17 had -CH₃ at the position a and -CH₂OH at the position b.

The compound of Example 18 had 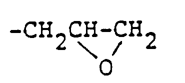
at the position a and 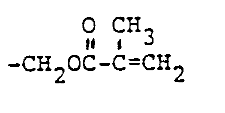
at the position b.

### (2) Results of analysis

Elemental analysis values:

| | C (%) | H (%) | N (%) |
|---|---|---|---|
| Example 15 | 82.9 (82.7) | 5.3 (5.5) | 5.4 (5.5) |
| Example 16 | 75.3 (75.3) | 4.7 (4.8) | 6.0 (6.1) |
| Example 17 | 77.4 (77.7) | 5.3 (5.4) | 6.2 (6.3) |
| Example 18 | 75.2 (75.3) | 5.2 (5.4) | 4.7 (5.0) |

Each value is a measured value, but each of the parenthesized values is a calculated value.

Laminated glass samples were prepared in the same manner as described in Example 9 using the compounds of Examples 15 through 17. Each of the laminated glass samples became green under irradiation with ultraviolet rays.

A glass sheet was prepared in the same manner as described in Example 7 using the compound of Example 18. The glass sheet became green under irradiation with ultraviolet rays.

### Example 19

### (1) Synthesis of 2,3,3-trimethylbenz(f)indolenine

Reaction was carried out in the same manner as described in (1) of Example 14 except that 2-naphthyl-hydrazine hydrochloride was used instead of 1-naphthyl-hydrazine hydrochloride

### (2) synthesis of spiro-oxazine of following formula (L):

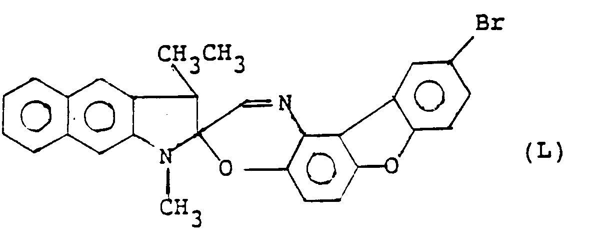

In 40 ml of ethanol were dissolved 6 g of the compound prepared in (1) above and 10 g of methyl tosylate, and reaction was carried out at the reflux temperature for 2 hours. The temperature of the reaction liquid was lowered to 50°C, and 2 g of triethylamine and 10 g of 1-nitroso-2-hydroxy-8-bromodibenzofuran were added to the reaction liquid. Reaction was carried out at the reflux temperature for 2 hours, and the subsequent procedures were the same as described in Example 1 and a light yellow crystal of the compound of the formula (L) was obtained.

### (3) Results of analysis

Elemental analysis values:

| | Found Value (%) | Calculated Value (%) |
|---|---|---|
| C | 67.3 | 67.6 |
| H | 4.0 | 4.2 |
| N | 5.7 | 5.6 |

### Example 20

### (1) Synthesis of 4,4-dimethyl-5-oxohexane-nitrile

A mixture of 40 g of 3-methyl-2-butanone and 2 g of Triton B was heated to 35°C, and 20 g of acrylonitrile was added to the mixture over a period of 1 hour. Then, the mixture was stirred for 18 hours, and hydrochloric acid was added to the mixture to make the mixture acidic and the mixture was extracted with ether.

### (2) Synthesis of compound of following formula (VI):

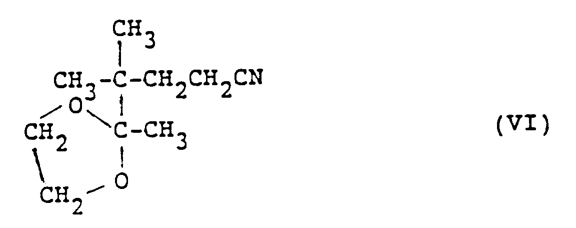

A mixture of 50 g of 4,4-dimethyl-5-oxohexane-nitrile, 6 g of ethylene glycol, 30 ml of petroleum ether and 0.3 g of p-toluenesulfonic acid monohydrate was reacted at the reflux temperature for 20 hours, and the reaction liquid was concentrated.

### (3) Synthesis of compound of following formula (VII):

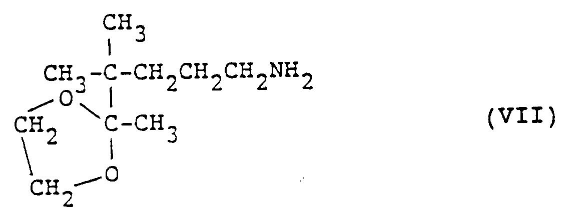

A solution of 20 g of the compound of the formula (VI) in 200 ml of ether was added dropwise to 20 g of lithium aluminum hydride and 300 ml of ether over a period of 30 minutes, and reaction was carried out at the reflux temperature for 2 hours and the reaction liquid was cooled. Water was added to the reaction liquid to decompose the excess of lithium aluminum hydride. The reaction mixture was filtered, and the filtrate was concentrated.

### (4) Synthesis of 2,3,3-trimethyl-3,4,5,6-tetrahydropyridine

To a solution of 20 g of the compound of the formula (VII) in 50 ml of ethanol was added 110 ml of 1N hydrochloric acid, and the mixture was heated and refluxed for 1 hour. Then, the reaction mixture was concentrated.

### (5) Synthesis of 1,3,3-trimethyl-2-methylene-piperidine

A mixture of 5 g of 2,3,3-trimethyl-3,4,5,6-tetrahydropyridine and 12 g of methyl tosylate was reacted at 100°C for 4 hours. The reaction mixture was cooled and extracted with water/chloroform. Then, 2 g of sodium hydroxide was added to the water layer and the mixture was stirred. The liquid was extracted with chloroform. The chloroform layer was concentrated to obtain a yellow liquid.

### (6) Synthesis of spiro-oxazine of following formula (M):

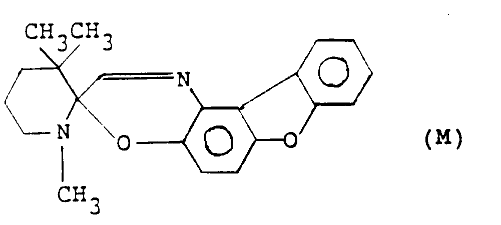

A milky white crystalline compound of the formula (M) was prepared in the same manner as described in Example 1 except that 1,3,3-trimethyl-2-methylene-piperidine was used instead of 1,3,3-trimethyl-2-methylene-indoline.

### (7) Results of Analysis

Elemental analysis values:

| | Found Value (%) | Calculated Value (%) |
|---|---|---|
| C | 75.5 | 75.5 |
| H | 6.6 | 6.6 |
| N | 8.4 | 8.4 |

### Example 21

### (1) synthesis of spiro-oxazine of following formula (N):

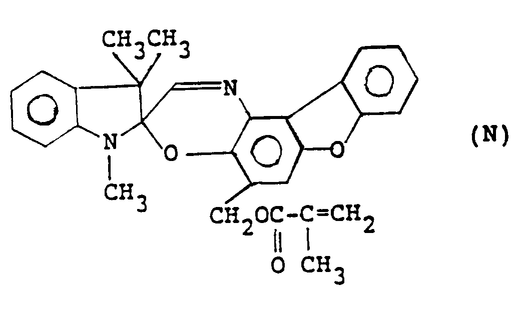

A white crystalline spiro-oxazine of the formula (N) was prepared in the same manner as described in Example 1 except that 1-nitroso-2-hydroxy-3-methacryloxymethyldibenzofuran was used instead of 1-nitroso-2-hydroxydibenzofuran.

### (2) Results of analysis

Elemental analysis values:

| | Found Value (%) | Calculated Value (%) |
|---|---|---|
| C | 74.4 | 74.7 |
| H | 5.5 | 5.6 |
| N | 6.1 | 6.0 |

IR: 1720 cm⁻¹

### (3) Application

A glass sheet was prepared in the same manner as described in Example 7 using the compound of the formula (N). When the glass sheet was irradiated with ultraviolet rays, the glass sheet became green, and when the light was removed and the glass sheet was allowed to stand in the dark, the glass sheet became colorless again.

### Example 22

### (1) Synthesis of spiro-oxazine of following formula (O):

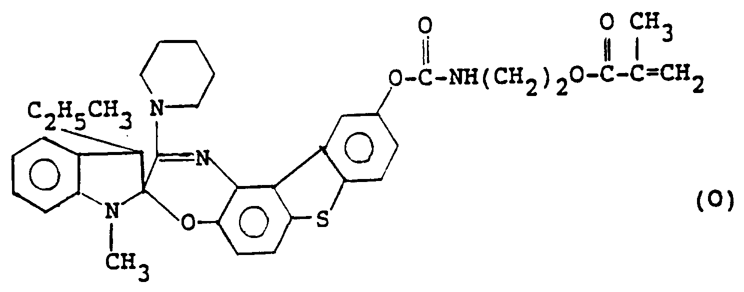

A solution comprising 15 g of 1-nitroso-2-hydroxy-8-(N-methacryloxyethyl)carbamoyloxydibenzothiophene, 20 g of piperidine and 100 ml of trichloroethylene was heated to the reflux temperature. Then, 9 g of 1,3-dimethyl-3-ethyl-2-methylene-indoline was added dropwise to the solution being refluxed over a period of 30 minutes. After the dropwise addition, reaction was carried out at the reflux temperature for 2 hours. After the reaction, the purification was carried out in the same manner as described in Example 1 to obtain a white crystalline spiro-oxazine of the formula (O).

### (2) Results of analysis

Elemental analysis values:

| | Found Value (%) | Calculated Value (%) |
|---|---|---|
| C | 68.1 | 68.1 |
| H | 6.2 | 6.1 |
| N | 8.5 | 8.6 |
| S | 4.6 | 4.9 |

IR: 3330 cm⁻¹, 1740 cm⁻¹, 1717 cm⁻¹, 1680 cm⁻¹, 1540 cm⁻¹

### (3) Application

When a solution of the compound of Example 22 in acetone was irradiated with ultraviolet rays, the solution became red, and when the light was removed and the solution was allowed to stand in the dark, the solution became colorless. Even when the above procedure was repeated 10 times, the photochromic characteristics were not changed at all.

### Example 23

Synthesis of spiro-oxazine of following formula (P): 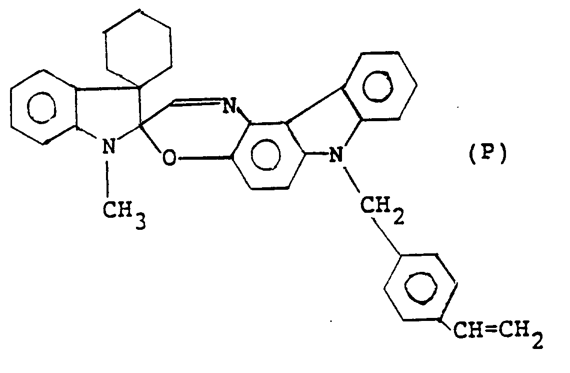
A milky white crystalline spiro-oxazine of the formula (P) was obtained in Example 1 except that 1-methyl-3-spirocyclohexyl-2-methylene-indoline was used instead of 1,3,3-trimethyl-2-methylene-indoline and 3-hydroxy-4-nitroso-9-(4-vinyl)benzylcarbazole was used instead of 1-nutroso-2-hydroxydibenzofuran.

### (2) Results of analysis

Elemental analysis values:

| | Found Value (%) | Calculated Value (%) |
|---|---|---|
| C | 82.5 | 82.6 |
| H | 6.0 | 6.3 |
| N | 7.9 | 8.0 |

### (3) Application

When a solution of the compound of Example 23 in acetone was irradiated with ultraviolet rays, the solution became red, and when the light was removed and the solution was allowed to stand in the dark, the solution became colorless again. Even when the above procedure was repeated 10 times, the photochromic characteristics were not changed at all.

### Example 24

### (1) Synthesis of spiro-oxazine of following formula (Q):

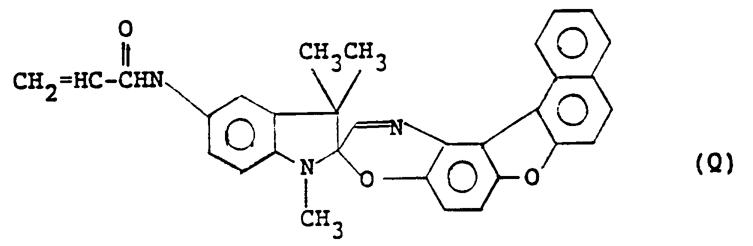

A light yellow crystalline compound of the formula (Q) was obtained in the same manner as described in Example 1 except that 1,3,3-trimethyl-2-methylene-5-acrylaminoindoline was used instead of 1,3,3-trimethyl-2-methylene-indoline and 8-hydroxy-9-nitroso(1,2-benzodiphenylene oxide) was used instead of 1-nitroso-2-hydroxydibenzofuran.

### (2) Results of analysis

Elemental analysis values:

| | Found Value (%) | Calculated Value (%) |
|---|---|---|
| C | 76.2 | 76.4 |
| H | 4.8 | 5.1 |
| N | 8.8 | 8.6 |

IR: 1675 cm⁻¹

### (3) Application

When a solution of the compound of Example 24 in acetone was irradiated with ultraviolet rays, the solution became green, and when the light was removed and the solution was allowed to stand in the dark, the solution became colorless. Even when the above procedure was repeated 10 times, the photochromic characteristics were not changed at all.

### Example 25

### (1) Synthesis of 2-hydroxy-9-methylcarbazole

To a mixture comprising 20 g of 2-hydroxycarbazole, 3 g of benzyltriethyl ammonium chloride, 70 ml of a 50% aqueous solution of sodium hydroxide and 10 ml of benzene was added dropwise 25 g of methyl iodide. After the dropwise addition, the mixture was stirred for 2 hours. The mixture was poured into hot water and the mixture was allowed to stand at room temperature overnight. The precipitated solid was recovered by filtration, washed with water and dried to obtain a yellowish brown solid of 2-hydroxy-9-methylcarbazole.

### (2) Synthesis of 1-nitroso-2-hydroxy-9-methylcarbazole

A red solid of 1-nitroso-2-hydroxy-9-methylcarbazole was prepared in the same manner as described in (1) of Example 1 except that the compound obtained in (1) above was used instead of 2-hydroxydibenzofuran.

### (3) Synthesis of spiro-oxazine of following formula (R):

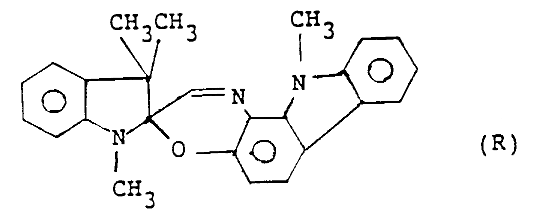

A greenish white crystalline spiro-oxazine of the formula (R) was prepared in the same manner as described in (2) of Example 1 except that the compound obtained in (2) above was used instead of 1-nitroso-2-hydroxydibenzofuran.

### (4) Results of analysis of compound of formula (R)

### Elemental analysis values:

| | Found Value (%) | Calculated Value (%) |
|---|---|---|
| C | 78.7 | 78.7 |
| H | 5.9 | 6.0 |
| N | 10.8 | 11.0 |

### (5) Application

When a solution of the compound of Example 25 in acetone was irradiated with ultraviolet rays, the solution became red, and when the light was removed and the solution was allowed to stand in the dark, the solution became colorless. Even when the above procedure was repeated 10 times, the photochromic characteristics were not changed at all.

### INDUSTRIAL APPLICABILITY

Since the spiro-oxazine compound of the present invention has connected benzene, heterocyclic 5-membered and benzene (or naphthalene) rings, represented by a dibenzofuran ring, the spiro-oxazine compound of the present invention has absorptions in both the short wavelength region (less than 550 nm) having a yellow chromophoric seed and the long wavelength region (larger than 600 nm) having a blue chromophoric seed in the visible ray region. Accordingly, a green chromophoric seed not obtainable by the conventional technique can be realized, and an abundance of hues can be selected. Therefore, the spiro-oxazine compound of the present invention is an epoch-making photochromic compound. Furthermore, the fatigue resistance is good.

Accordingly, the compound of the present invention is valuable as an optical element such as in sunglasses, ski goggles or protective glass lenses, or a material for curtaining, clothing, automobile windows, toys, writing tools, or rewritable optical disks.

## Claims

1. A spiro-oxazine compound represented by the following general formula (A) or (A'): 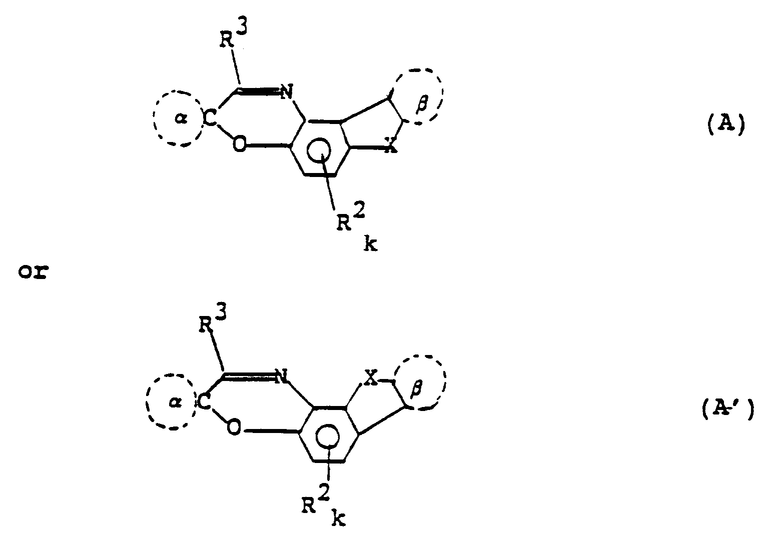 wherein:
the α ring is a ring selected from a 5-membered ring having one nitrogen atom, a 5-membered ring having one nitrogen atom and connected to a benzene ring or naphthalene ring and a 6-membered ring having one nitrogen atom, with the proviso that the nitrogen atom in the α ring is bonded to an organic group R⁰, expressed as > N-R⁰ [in which R⁰ is a substituent selected from an alkyl group having 1 to 20 carbon atoms, an alkenyl group having 2 to 20 carbon atoms, an aralkyl group having 7 to 20 carbon atoms and an aryl group having 6 to 19 carbon atoms, each of which groups of R⁰ is optionally substituted by a hydroxyl group, an amino, dibenzylamino or 2-methacryloxyethyl amino group, a methoxy, ethoxy or propoxy group, an acetoxy, benzoyloxy, acryloxy or methacryloxy group, a methyl, ethyl, trifluoromethyl or butyl group, a benzyl or 4-(2,3-epoxypropyl)phenethyl group, a phenyl or styryl group, a halogen atom, a cyano group, a carboxyl group, a nitro group, an acetyl group, a methacryl group, an ethoxycarbonyl or 3,4-epoxybutyloxycarbonyl group, a carbamoyl, N-phenylcarbamoyl or N-(2-methacryloxy)ethylcarbamoyl group, a carbamoyloxy or [N-(acryloxy)propylcarbamoyl]oxy group or a sulphonic acid group (optionally in the form of free sulphonic acid or a metal salt thereof)],
the β ring is a benzene or naphthalene ring,
X is a radical selected from O, S, Se and N-R¹ (in which R¹ is a substituent selected from a hydrogen atom, an alkyl group having 1 to 20 carbon atoms, an alkenyl group having 2 to 20 carbon atoms, an aralkyl group having 7 to 20 carbon atoms, an aryl group having 6 to 19 carbon atoms (which alkyl, alkenyl, aralkyl or aryl group is optionally substituted by any of the optional substituents of R⁰ defined above) and an acyl group having 2 to 20 carbon atoms),
each of R² and R³ is a substituent selected from a hydroxyl group, an amino, dimethylamino, dibenzylamino or 2-methacryloxyethyl amino group, an alkoxy group having 1 to 20 carbon atoms, an aralkoxy group having 7 to 15 carbon atoms, an aryloxy group having 6 to 14 carbon atoms, an acyloxy group having 2 to 20 carbon atoms, an alkyl group having 1 to 20 carbon atoms, an alkenyl group having 2 to 20 carbon atoms, an aralkyl group having 7 to 15 carbon atoms, an aryl group having 6 to 14 carbon atoms, a halogen atom, a cyano group, a carboxyl group, a nitro group (not included in R³), an acyl group having 2 to 20 carbon atoms, an alkoxycarbonyl group having 2 to 20 carbon atoms, a carbamoyl, N-phenylcarbamoyl or N-(2-methacryloxy)ethyl-carbamoyl, a carbamoyloxy, [N-(3-acryloxy)propyl-carbamoyl]oxy or [N-(3-methacryloxy)propyl-carbamoyl]oxy group and a sulphonic acid group (optionally in the form of a free sulphonic acid group or a metal salt thereof and not included in R³), with the proviso that a hydrogen atom is included in R³, k is 0 or an integer of up to 2, and when k is 2 each R² may be the same as or different from the other.

2. A spiro-oxazine compound according to claim 1, which is represented by the following formula (B) or (B'): 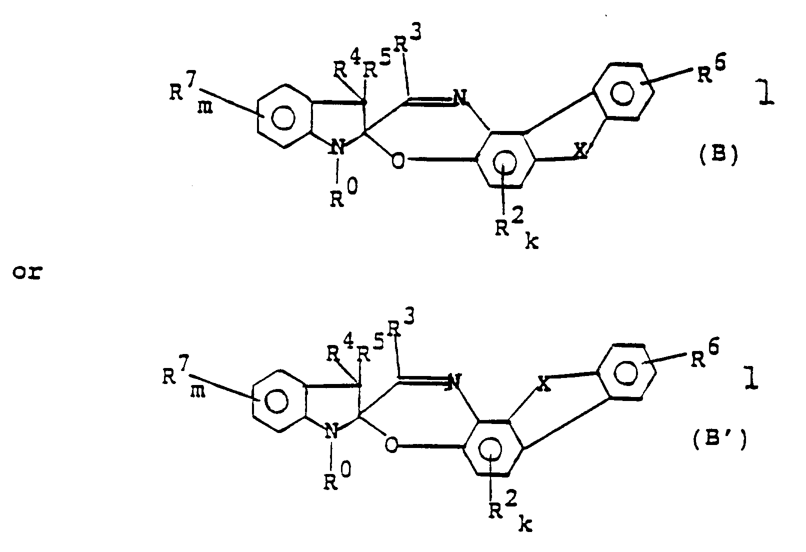 wherein R⁰, R², R³, X and k are as defined in claim 1, each of R⁴ and R⁵ independently is a substituent selected from an alkyl group having 1 to 20 carbon atoms, an aralkyl group having 7 to 20 carbon atoms and an aryl group having 6 to 19 carbon atoms, which alkyl, aralkyl or aryl group is optionally substituted by any of the optional substituents of R⁰ defined in claim 1, or R⁴ and R⁵ together form a cycloalkyl group having 3 to 10 carbon atoms with the carbon atom at the 3'-position, each of R⁶ and R⁷, which may be the same as or different from the other, is a substituent selected from any of the groups of R² as defined in claim 1, each of l and m is 0 or an integer up to 4, and when l or m is 2 or more each of R⁶ and R⁷ respectively may be the same as or different from each other.

3. A spiro-oxazine compound according to claim 1, which is represented by the following general formula (C) or (C'): 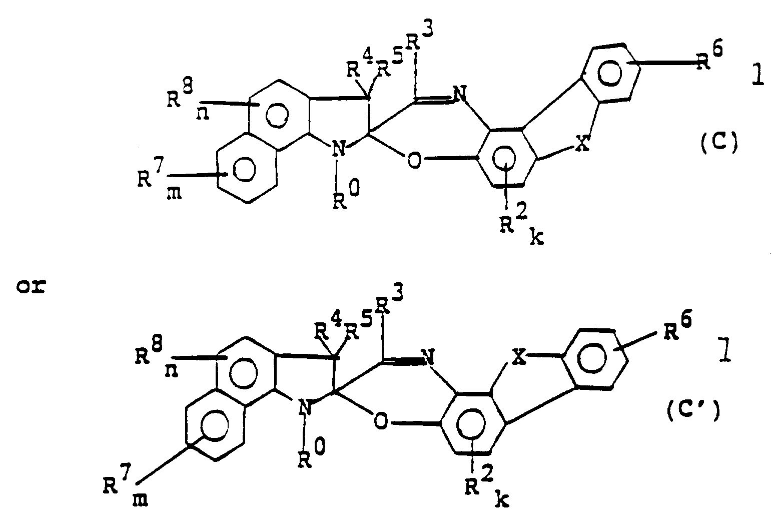 wherein R⁰, R², R³, X and k are as defined in claim 1, R⁴, R⁵, R⁶, R⁷, l and m are as defined in claim 2, R⁸ is a substituent selected from any of the groups of R² as defined in claim 1, n is 0 or an integer up to 2, and when n is 2 each R⁸ may be the same as or different from the other.

4. A spiro-oxazine compound according to claim 1, which is represented by the following general formula (D) or (D'): 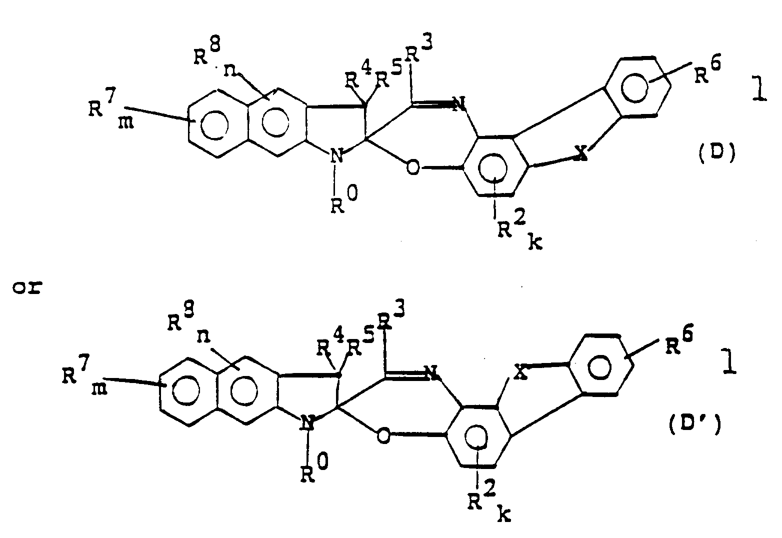 wherein R⁰, R², R³, and X and k are as defined in claim 1, R⁴, R⁵, R⁶, R⁷, l and m are as defined in claim 2, and R⁸ and n are as defined in claim 3.

5. A spiro-oxazine compound according to claim 1, which is represented by the following general formula (E) or (E'): 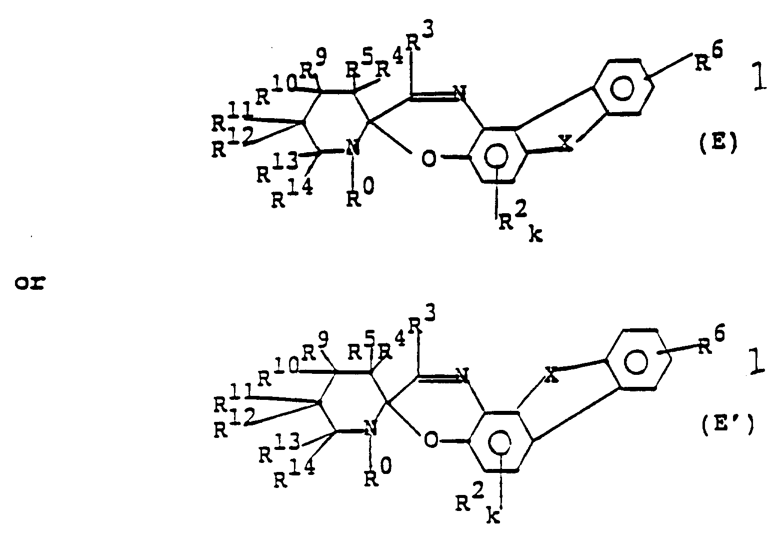 wherein R⁰, R², R³, X and k are as defined in claim 1, R⁴, R⁵, R⁶ and l are as defined in claim 2, and each of R⁹, R¹⁰, R¹¹, R¹², R¹³ and R¹⁴ is a substituent selected from a hydrogen atom, an alkyl group having 1 to 20 carbon atoms, an aralkyl group having 7 to 20 carbon atoms and an aryl group having 6 to 19 carbon atoms.

6. A spiro-oxazine compound according to claim 1, which is represented by the following general formula (F) or (F'): 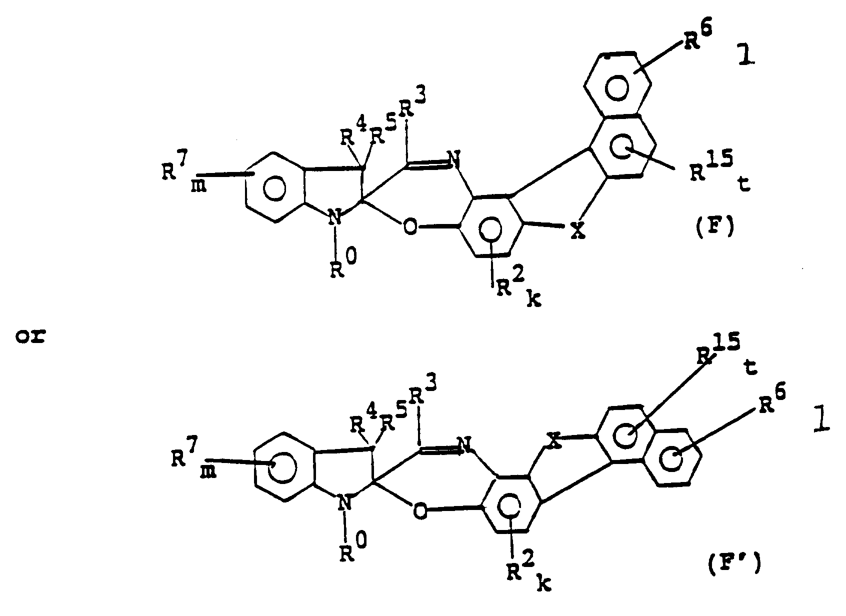 wherein R⁰, R², R³, X and k are as defined in claim 1, R⁴, R⁵, R⁶, R⁷, l and m are as defined in claim 2, R¹⁵ is a substituent selected from a hydroxyl group, an amino group, an alkyl group having 1 to 20 carbon atoms, an alkoxy group having 1 to 20 carbon atoms, an aralkoxy group having 7 to 15 carbon atoms, an aryloxy group having 6 to 14 carbon atoms, an acyloxy group having 2 to 20 carbon atoms, an alkenyl group having 2 to 20 carbon atoms, an aralkyl group having 7 to 15 carbon atoms, an aryl group having 6 to 14 carbon atoms, a halogen atom, a cyano group, a carboxyl group, a nitro group, an alkoxycarbonyl group having 2 to 20 carbon atoms, a carbamoyl group, a carbamoyloxy group and a sulphonic acid group, and t is 0 or an integer up to 2.

## Patentansprüche

1. Spirooxazin-Verbindung, die durch folgende allgemeine Formel (A) oder (A') dargestellt wird: 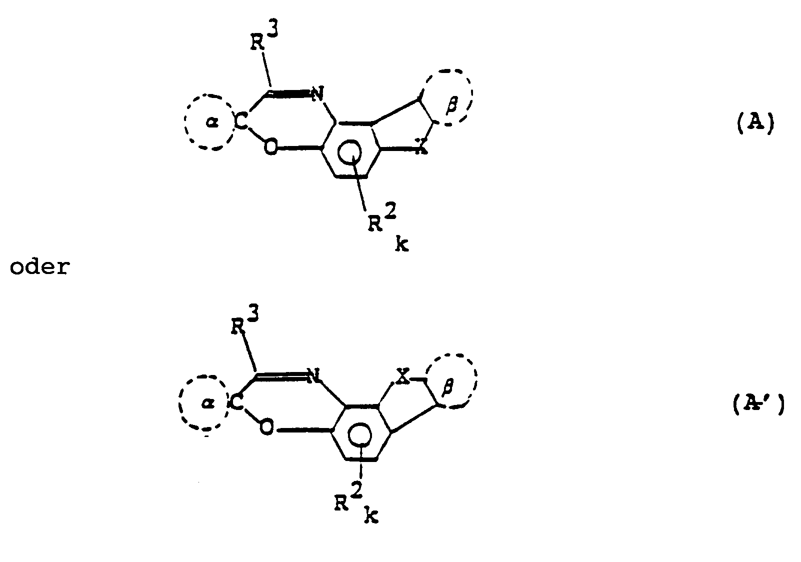 worin:
der α-Ring ein Ring ist, der ausgewählt ist aus einem 5-gliedrigen Ring mit einem Stickstoffatom, einem 5-gliedrigen Ring mit einem Stickstoffatom, der mit einem Benzolring oder einem Naphthalinring verbunden ist, und einem 6-gliedrigen Ring mit einem Stickstoffatom, vorausgesetzt, daß das Stickstoffatom im α-Ring mit der organischen Gruppe R⁰ verbunden ist, ausgedrückt als >N-R⁰ [worin R⁰ ein Substituent ist, ausgewählt aus einer Alkylgruppe mit 1 bis 20 Kohlenstoffatomen, einer Alkenylgruppe mit 2 bis 20 Kohlenstoffatomen, einer Aralkylgruppe mit 7 bis 20 Kohlenstoffatomen und einer Arylgruppe mit 6 bis 19 Kohlenstoffatomen, wobei jede Gruppe von R⁰ gegebenenfalls substituiert ist mit einer Hydroxylgruppe, einer Amino-, Dibenzylamino- oder 2-Methacryloxyethylaminogruppe, einer Methoxy-, Ethoxy- oder Propoxygruppe, einer Acetoxy-, Benzoyloxy-, Acryloxy- oder Methacryloxygruppe, einer Methyl-, Ethyl-, Trifluormethyl- oder Butylgruppe, einer Benzyl- oder 4-(2,3-Epoxypropyl)phenethylgruppe, einer Phenyl- oder Styrylgruppe, einem Halogenatom, einer Cyanogruppe, einer Carboxylgruppe, einer Nitrogruppe, einer Acetylgruppe, einer Methacrylgruppe, einer Ethoxycarbonyl- oder 3,4-Epoxybutyloxycarbonylgruppe, einer Carbamoyl-, N-Phenylcarbamoyl- oder N-(2-Methacryloxy)ethylcarbamoylgruppe, einer Carbamoyloxy- oder [N-(Acryloxy)propylcarbamoyl]oxygruppe oder einer Sulfonsäuregruppe (gegebenenfalls in Form der freien Sulfonsäure oder eines Metallsalzes davon)],
der β-Ring ein Benzol- oder Naphthalinring ist,
X ein Radikal ist, ausgewählt aus O, S, Se und N-R¹ (worin R¹ ein Substituent ist, der ausgewählt ist aus einem Wasserstoffatom, einer Alkylgruppe mit 1 bis 20 Kohlenstoffatomen, einer Alkenylgruppe mit 2 bis 20 Kohlenstoffatomen, einer Aralkylgruppe mit 7 bis 20 Kohlenstoffatomen, einer Arylgruppe mit 6 bis 19 Kohlenstoffatomen (wobei die Alkyl-, Alkenyl-, Aralkyl- oder Arylgruppe gegebenenfalls mit einem der oben definierten wahlfreien Subtituenten von R⁰ substituiert ist) und einer Acylgruppe mit 2 bis 20 Kohlenstoffatomen,
jeder R² und R³ ein Substituent ist, der ausgewählt ist aus einer Hydroxylgruppe, einer Amino-, Dimethylamino-, Dibenzylamino- oder 2-Methacryloxyethylaminogruppe, einer Alkoxygruppe mit 1 bis 20 Kohlenstoffatomen, einer Aralkoxygruppe mit 7 bis 15 Kohlenstoffatomen, einer Aryloxygruppe mit 6 bis 14 Kohlenstoffatomen, einer Acyloxygruppe mit 2 bis 20 Kohlenstoffatomen, einer Alkylgruppe mit 1 bis 20 Kohlenstoffatomen, einer Alkenylgruppe mit 2 bis 20 Kohlenstoffatomen, einer Aralkylgruppe mit 7 bis 15 Kohlenstoffatomen, einer Arylgruppe mit 6 bis 14 Kohlenstoffatomen, einem Halogenatom, einer Cyanogruppe, einer Carboxylgruppe, einer Nitrogruppe (in R³ nicht enthalten), einer Acylgruppe mit 2 bis 20 Kohlenstoffatomen, einer Alkoxycarbonylgruppe mit 2 bis 20 Kohlenstoffatomen, einer Carbamoyl-, N-Phenylcarbamoyl- oder N-(2-Methacryloxy)ethylcarbamoyl-, einer Carbamoyloxy-, [N-(3-Acryloxy)propylcarbamoyl]oxy- oder [N-(3-Methacryloxy)propylcarbamoyl]oxygruppe und einer Sulfonsäuregruppe (gegebenfalls in Form der freien Sulfonsäuregruppe oder eines Metallsalzes davon und in R³ nicht enthalten), vorausgesetzt, daß das Wasserstoffatom in R³ enthalten ist, k 0 oder eine ganze Zahl bis 2 ist, und falls k 2 ist, jedes R² gleich oder voneinander verschieden sein kann.

2. Spirooxazin-Verbindung nach Anspruch 1, die durch folgende Formel (B) oder (B') dargestellt wird: 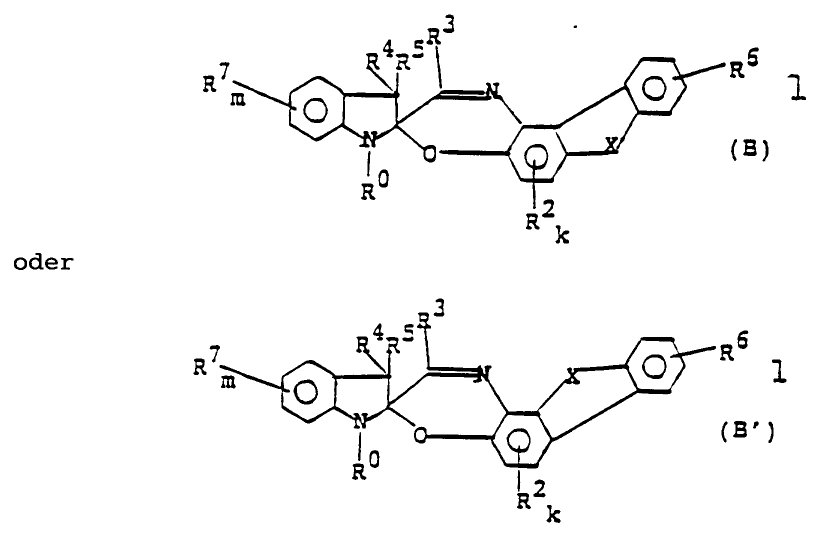 worin R⁰, R², R³, X und k wie in Anspruch 1 definiert sind, jedes R⁴ und R⁵ unabhängig voneinander ein Substituent ist, der ausgewählt ist aus einer Alkylgruppe mit 1 bis 20 Kohlenstoffatomen, einer Aralkylgruppe mit 7 bis 20 Kohlenstoffatomen, einer Arylgruppe mit 6 bis 19 Kohlenstoffatomen, wobei die Alkyl-, Aralkyl- oder Arylgruppe gegebenenfalls mit einem der wahlfreien Substituenten von R⁰ substituiert ist, die in Anspruch 1 definiert sind, oder R⁴ und R⁵ zusammen eine Cycloalkylgruppe mit 3 bis 10 Kohlenstoffatomen mit dem Kohlenstoffatom in der 3'-Position bilden, jeder R⁶ und R⁷, die gleich oder verschieden voneinander sein können, ein Substituent ist, der aus jeder der Gruppen von R² ausgewählt ist, die in Anspruch 1 definiert sind, jedes l und m 0 oder eine ganze Zahl bis 4 ist, und falls l oder m 2 oder mehr ist, jeder R⁶ bzw. R⁷ gleich oder verschieden voneinander sein kann.

3. Spirooxazin-Verbindung nach Anspruch 1, die durch folgende allgemeine Formel (C) oder (C') dargestellt wird: 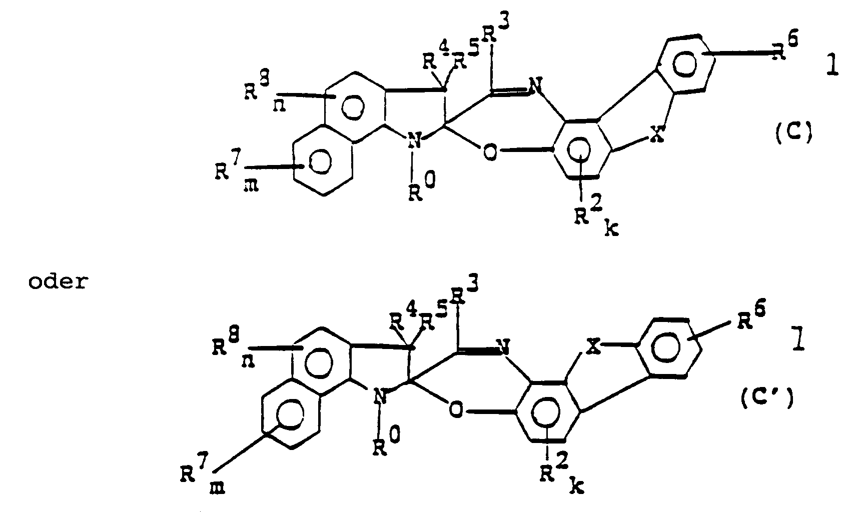 worin R⁰, R², R³, X und k wie in Anspruch 1 definiert sind, R⁴, R⁵, R⁶, R⁷, l und m wie in Anspruch 2 definiert sind, und R⁸ ein Substituent ist, der aus jeder der Gruppen von R² ausgewählt ist, die in Anspruch 1 definiert sind, n 0 oder eine ganze Zahl bis 2 ist, und falls n 2 ist, jedes R⁸ gleich oder voneinander verschieden sein kann.

4. Spirooxazin-Verbindung nach Anspruch 1, die durch folgende allgemeine Formel (D) oder (D') dargestellt wird: 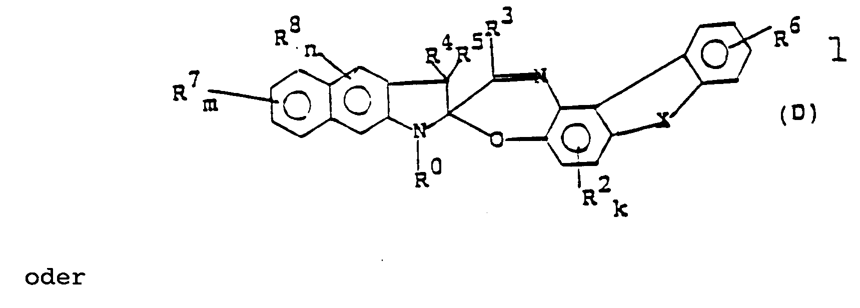 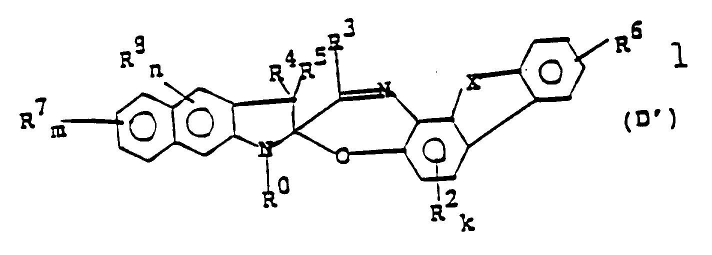 worin R⁰, R², R³ und X und k wie in Anspruch 1 definiert sind, R⁴, R⁵, R⁶, R⁷, l und m wie in Anspruch 2 definiert sind, und R⁸ und n wie in Anspruch 3 definiert sind.

5. Spirooxazin-Verbindung nach Anspruch 1, die durch folgende allgemeine Formel (E) oder (E') dargestellt wird: 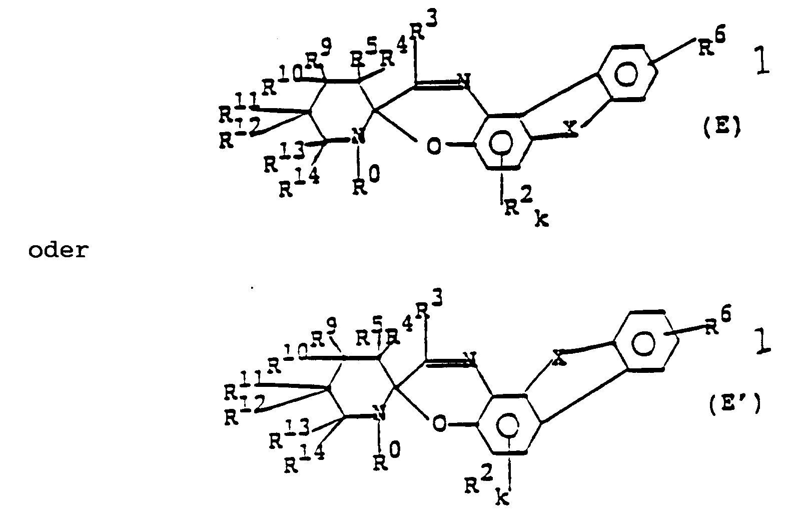 worin R⁰, R², R³, X und k wie in Anspruch 1 definiert sind, R⁴, R⁵, R⁶ und l wie in Anspruch 2 definiert sind, jeder R⁹, R¹⁰, R¹¹, R¹², R¹³ und R¹⁴ ein Substituent ist, der ausgewählt ist aus einem Wasserstoffatom, einer Alkylgruppe mit 1 bis 20 Kohlenstoffatomen, einer Aralkylgruppe mit 7 bis 20 Kohlenstoffatomen und einer Arylgruppe mit 6 bis 19 Kohlenstoffatomen.

6. Spirooxazin-Verbindung nach Anspruch 1, die durch folgende allgemeine Formel (F) oder (F') dargestellt wird: 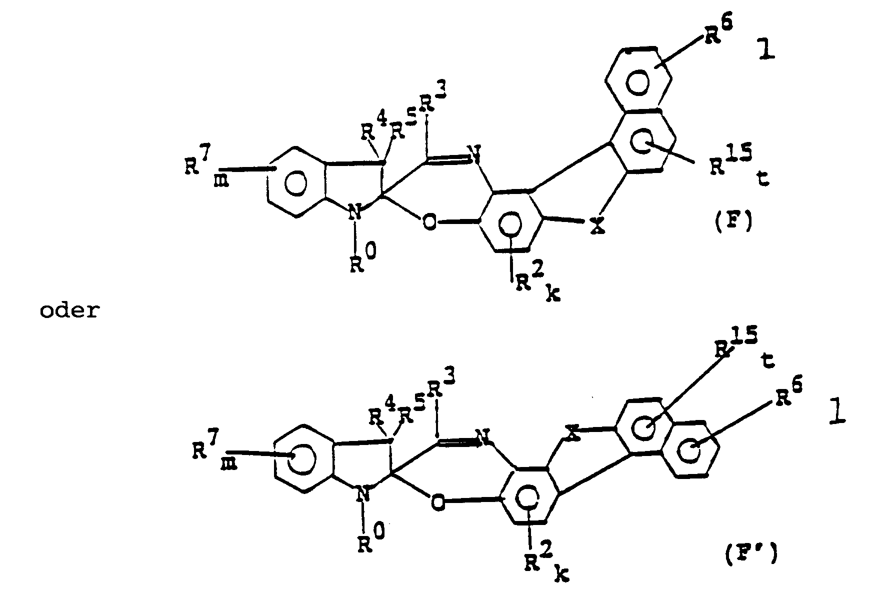 worin R⁰, R², R³, X und k wie in Anspruch 1 definiert sind, R⁴, R^{5,} R⁶, R⁷, l und m wie in Anspruch 2 definiert sind, R¹⁵ ein Substituent ist, der ausgewählt ist aus einer Hydroxylgruppe, einer Aminogruppe, einer Alkylgruppe mit 1 bis 20 Kohlenstoffatomen, einer Alkoxygruppe mit 1 bis 20 Kohlenstoffatomen, einer Aralkoxygruppe mit 7 bis 15 Kohlenstoffatomen, einer Aryloxygruppe mit 6 bis 14 Kohlenstoffatomen, einer Acyloxygruppe mit 2 bis 20 Kohlenstoffatomen, einer Alkenylgruppe mit 2 bis 20 Kohlenstoffatomen, einer Aralkylgruppe mit 7 bis 15 Kohlenstoffatomen, einer Arylgruppe mit 6 bis 14 Kohlenstoffatomen, einem Halogenatom, einer Cyanogruppe, einer Carboxylgruppe, einer Nitrogruppe, einer Alkoxycarbonylgruppe mit 2 bis 20 Kohlenstoffatomen, einer Carbamoylgruppe, einer Carbamoyloxygruppe und einer Sulfonsäuregruppe, und t 0 oder eine ganze Zahl bis 2 ist.

## Revendications

1. Spirooxazine représentée par les formules générales (A) ou (A') suivantes : 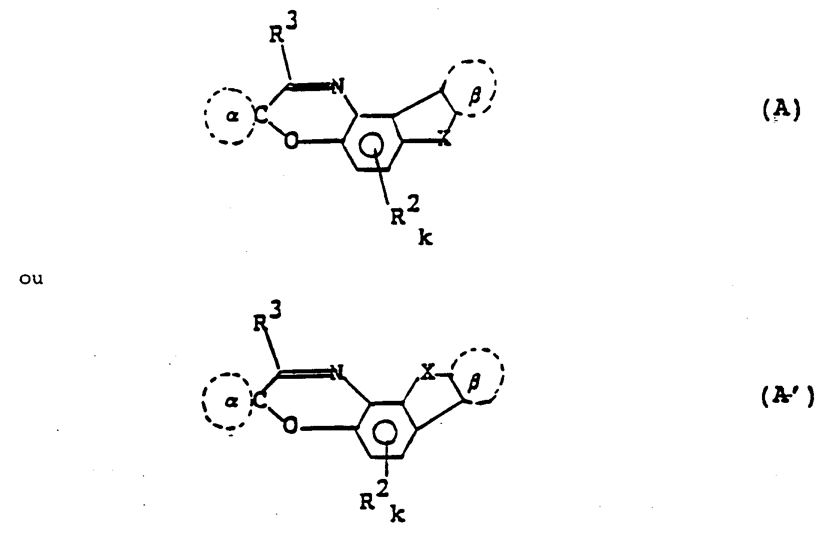 où
le cycle α est un cycle choisi parmi un cycle à 5 chaînons ayant un atome d'azote, un cycle à 5 chaînons ayant un atome d'azote et réuni à un cycle benzène ou à un cycle naphtalène, et un cycle à 6 chaînons ayant un atome d'azote, sous réserve que l'atome d'azote du cycle α soit lié à un groupe organique R⁰, exprimé comme >N-R⁰ [où R⁰ est un substituant choisi parmi un groupe alkyle ayant 1 à 20 atomes de carbone, un groupe alcényle ayant 2 à 20 atomes de carbone, un groupe aralkyle ayant 7 à 20 atomes de carbone et un groupe aryle ayant 6 à 19 atomes de carbone, chacun de ces groupes R⁰ étant éventuellement substitué par un groupe hydroxy, un groupe amino, dibenzylamino ou 2-méthacryloxyéthylamino, un groupe méthoxy, éthoxy ou propoxy, un groupe acétoxy, benzoyloxy, acryloxy ou méthacryloxy, un groupe méthyle, éthyle, trifluorométhyle ou butyle, un groupe benzyle ou 4-(2,3-époxypropyl)phénéthyle, un groupe phényle ou styryle, un atome d'halogène, un groupe cyano, un groupe carboxy, un groupe nitro, un groupe acétyle, un groupe méthacryle, un groupe éthoxycarbonyle ou 3,4-époxybutyloxycarbonyle, un groupe carbamoyle, N-phénylcarbamoyle ou N-(2-méthacryloxy)éthylcarbamoyle, un groupe carbamoyloxy ou [N-(acryloxy)propylcarbamoyl]oxy ou un groupe acide sulfonique (sous forme de l'acide sulfonique libre ou d'un de ses sels métalliques)] ; le cycle β est un cycle benzène ou naphtalène,
X est un radical choisi parmi O, S, Se et N-R¹ (où R¹ est un substituant choisi parmi un atome d'hydrogène, un groupe alkyle ayant 1 à 20 atomes de carbone, un groupe alcényle ayant 2 à 20 atomes de carbone, un groupe aralkyle ayant 7 à 20 atomes de carbone, un groupe aryle ayant 6 à 19 atomes de carbone (où le groupe alkyle, alcényle, aralkyle ou aryle est éventuellement substitué par l'un quelconque des substituants éventuels de R⁰ définis ci-dessus) et un groupe acyle ayant 2 à 20 atomes de carbone),
chacun de R² et R³ est un substituant choisi parmi un groupe hydroxy, un groupe amino, diméthylamino, dibenzylamino ou 2-méthacryloxyéthylamino, un groupe alcoxy ayant 1 à 20 atomes de carbone, un groupe aralcoxy ayant 7 à 15 atomes de carbone, un groupe aryloxy ayant 6 à 14 atomes de carbone, un groupe acyloxy ayant 2 à 20 atomes de carbone, un groupe alkyle ayant 1 à 20 atomes de carbone, un groupe alcényle ayant 2 à 20 atomes de carbone, un groupe aralkyle ayant 7 à 15 atomes de carbone, un groupe aryle ayant 6 à 14 atomes de carbone, un atome d'halogène, un groupe cyano, un groupe carboxy, un groupe nitro (non compris dans R³), un groupe acyle ayant 2 à 20 atomes de carbone, un groupe alcoxycarbonyle ayant 2 à 20 atomes de carbone, un groupe carbamoyle, N-phénylcarbamoyle ou N-(2-méthacryloxy)éthylcarbamoyle, un groupe carbamoyloxy, [N-(3-acryloxy)propylcarbamoyl]oxy ou [N-(3-méthacryloxy)propylcarbamoyl]oxy et un groupe acide sulfonique (sous forme d'un groupe acide sulfonique libre ou d'un sel métallique de celui-ci et non compris dans R³), sous réserve qu'un atome d'hydrogène soit compris dans R³, k est 0 ou un entier jusqu'à 2 et, lorsque k est 2, les R² peuvent être identiques ou différents.

2. Spirooxazine selon la revendication 1, représentée par les formules (B) ou (B') suivantes : 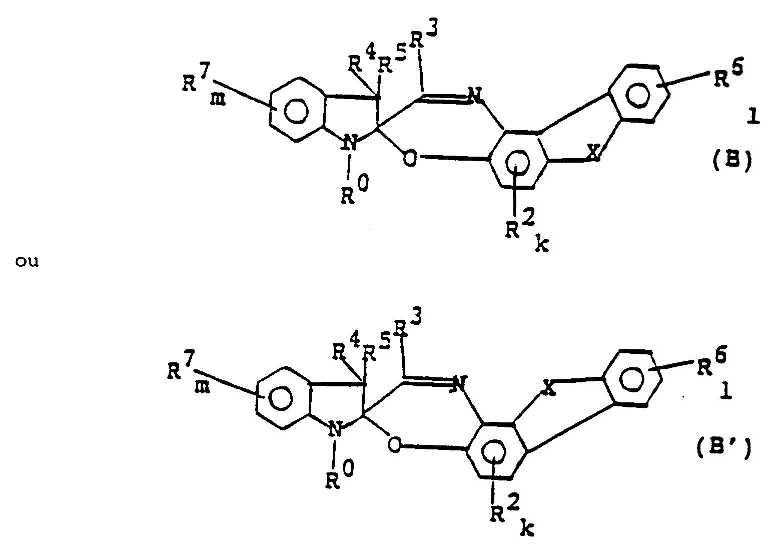 où R⁰, R², R³, X et k sont tels que définis dans la revendication 1, chacun de R⁴ et R⁵ indépendamment est un substituant choisi parmi un groupe alkyle ayant 1 à 20 atomes de carbone, un groupe aralkyle ayant 7 à 20 atomes de carbone et un groupe aryle ayant 6 à 19 atomes de carbone où le groupe alkyle, aralkyle ou aryle est éventuellement substitué par l'un quelconque des substituants éventuels de R⁰ définis dans la revendication 1, ou R⁴ et R⁵ forment ensemble un groupe cycloalkyle ayant 3 à 10 atomes de carbone avec l'atome de carbone dans la position 3', chacun de R⁶ et R⁷, qui peuvent être identiques ou différents, est un substituant choisi parmi l'un quelconque des groupes de R² tel que définis dans la revendication 1, chacun de l et m est 0 ou un entier jusqu'à 4, et, lorsque l ou m est 2 ou plus, les R⁶ et R⁷ peuvent respectivement chacun être identiques ou différents.

3. Spirooxazine selon la revendication 1 qui est représentée par les formules générales (C) ou (C') suivantes : 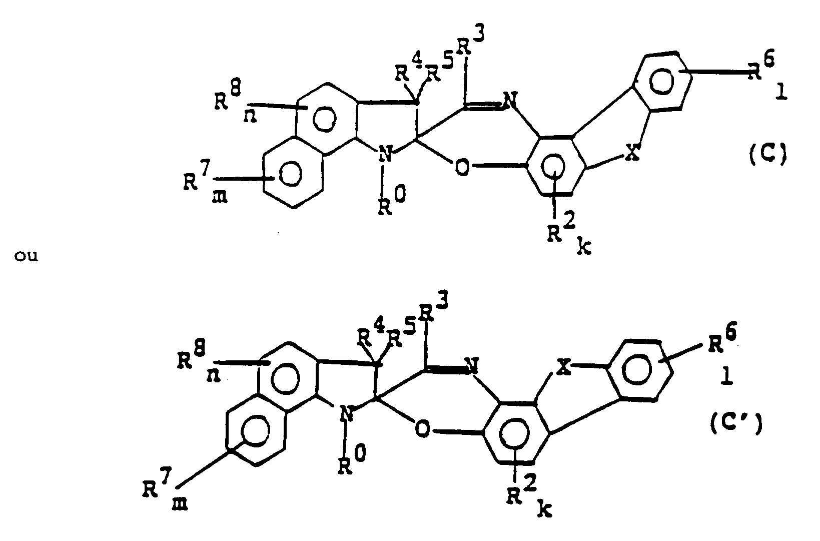 où R⁰, R², R³, X et k sont tels que définis dans la revendication 1, R⁴, R⁵, R⁶, R⁷, l et m sont tels que définis dans la revendication 2, R⁸ est un substituant choisi parmi l'un quelconque des groupes de R² tels que définis dans la revendication 1, n est 0 ou un entier jusqu'à 2 et, lorsque n est 2, les R⁸ peuvent être identiques ou différents.

4. Spirooxazine selon la revendication 1 qui est représentée par les formules générales (D) ou (D') suivantes : 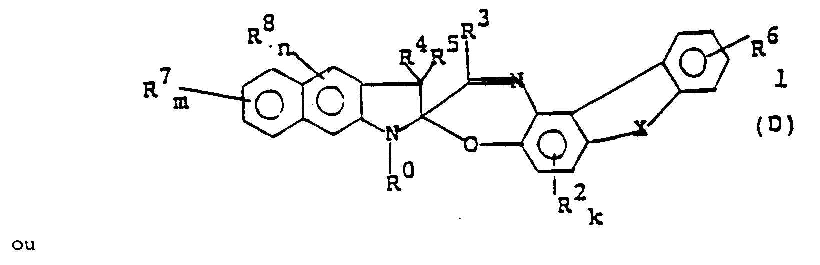 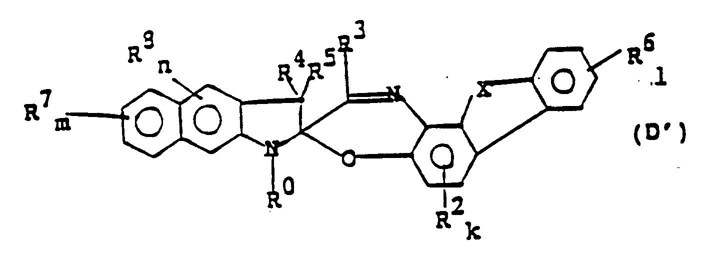 où R⁰, R², R³, X et k sont tels que définis dans la revendication 1, R⁴, R⁵, R⁶, R⁷, l et m sont tels que définis dans la revendication 2 et R⁸ et n sont tels que définis dans la revendication 3.

5. Spirooxazine selon la revendication 1 qui est représentée par les formules générales (E) ou (E') suivantes : 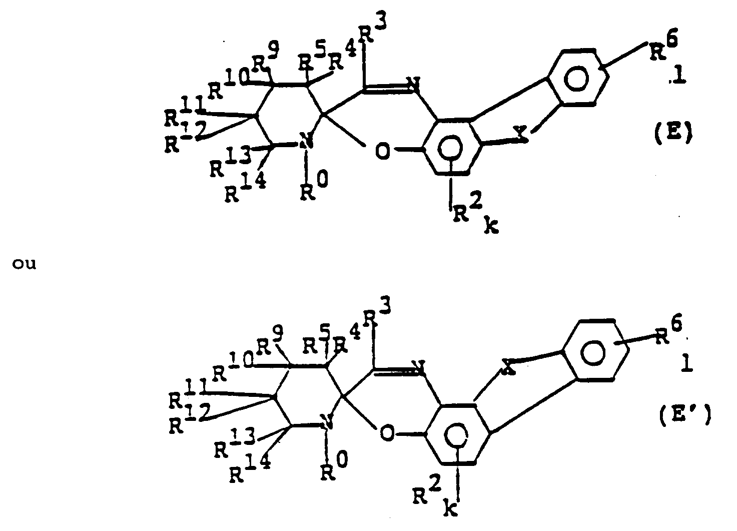 où R⁰, R², R³, X et k sont tels que définis dans la revendication 1, R⁴, R⁵, R⁶ et l sont tels que définis dans la revendication 2 et chacun de R⁹, R¹⁰, R¹¹, R¹², R¹³ et R¹⁴ est un substituant choisi parmi un atome d'hydrogène, un groupe alkyle ayant 1 à 20 atomes de carbone, un groupe aralkyle ayant 7 à 20 atomes de carbone et un groupe aryle ayant 6 à 19 atomes de carbone.

6. Spirooxazine selon la revendication 1 qui est représentée par les formules générales (F) ou (F') : 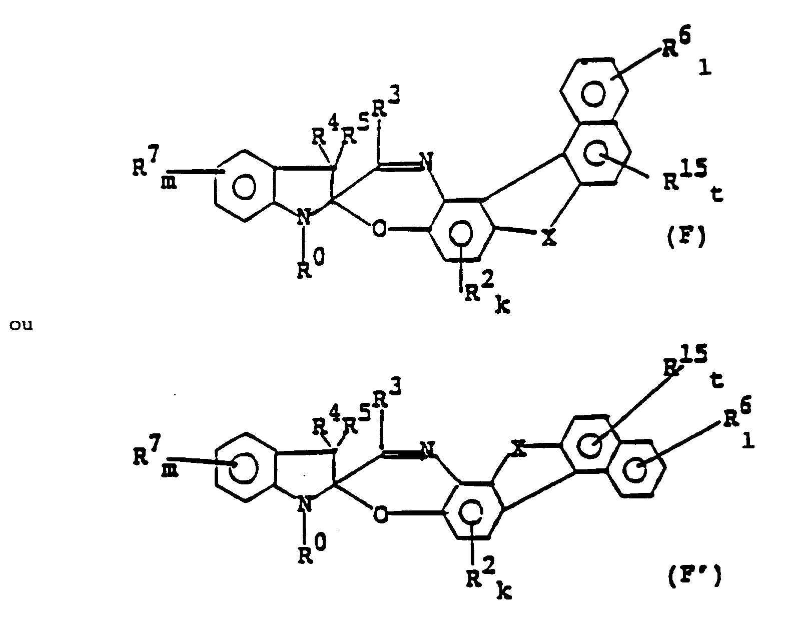 où R⁰, R², R³, X et k sont tels que définis dans la revendication 1, R⁴, R⁵, R⁶, R⁷, l et m sont tels que définis dans la revendication 2, R¹⁵ est un substituant choisi parmi un groupe hydroxy, un groupe amino, un groupe alkyle ayant 1 à 20 atomes de carbone, un groupe alcoxy ayant 1 à 20 atomes de carbone, un groupe aralcoxy ayant 7 à 15 atomes de carbone, un groupe aryloxy ayant 6 à 14 atomes de carbone, un groupe acyloxy ayant 2 à 20 atomes de carbone, un groupe alcényle ayant 2 à 20 atomes de carbone, un groupe aralkyle ayant 7 à 15 atomes de carbone, un groupe aryle ayant 6 à 14 atomes de carbone, un atome d'halogène, un groupe cyano, un groupe carboxy, un groupe nitro, un groupe alcoxycarbonyle ayant 2 à 20 atomes de carbone, un groupe carbamoyle, un groupe carbamoyloxy et un groupe acide sulfonique et t est 0 ou un entier jusqu'à 2.
